Europäisches Patentamt

European Patent Office

Office européen des brevets

(19)

(11) **EP 0 630 255 B1**

(12) **EUROPEAN PATENT SPECIFICATION**

(45) Date of publication and mention
of the grant of the patent:
**18.12.2002 Bulletin 2002/51**

(51) Int Cl.[7]: **C07K 7/04**, A61K 39/00,
A61K 39/385

(21) Application number: **92911380.1**

(22) Date of filing: **23.04.1992**

(86) International application number:
**PCT/US92/03391**

(87) International publication number:
**WO 92/018150 (29.10.1992 Gazette 1992/27)**

(54) **MHC CONJUGATES USEFUL IN AMELIORATING AUTOIMMUNITY**

MHC-Konjugate zur Verbesserung der Autoimmunität.

CONJUGUES CMH PERMETTANT DE TRAITER L'AUTO-IMMUNITE

(84) Designated Contracting States:
**CH DE FR GB IT LI**

(30) Priority: **23.04.1991 US 690840**
**14.04.1992 US 869293 P**

(43) Date of publication of application:
**28.12.1994 Bulletin 1994/52**

(73) Proprietor: **ANERGEN, INC.**
**Redwood City, CA 94063 (US)**

(72) Inventors:
• **CLARK, Brian, R.**
**Redwood City, CA 94061 (US)**
• **SHARMA, Somesh, D.**
**Los Altos, CA 94022 (US)**
• **LERCH, Bernard, L.**
**Palo Alto, CA 94303 (US)**

(74) Representative: **Armitage, Ian Michael et al**
**MEWBURN ELLIS**
**York House**
**23 Kingsway**
**London WC2B 6HP (GB)**

(56) References cited:
EP-A- 0 432 691          WO-A-89/12459
WO-A-93/09810          US-A- 4 478 823

• PROCEEDINGS OF THE NATIONAL ACADEMY
OF SCIENCES OF USA, vol.87, no.4, 1990 pages
1337 - 1341 KUMAR V., ET AL.

• Immunol. Res., Volume 6, issued 1987,
MARGULIES et al., "Engineering Soluble
Molecules: Why and How", pages 101-116, see
entire article.
• J. Exp. Med., Volume 150, issued November
1979, BINZ et al., "Binding of Purified Major
Histocompatability Complex Polypeptide
Chains onto Isolated T-cell Receptors", pages
1084-1097, see entire article.
• Eur. J. Immunol., Volume 19, 1989, VILLAR et al.,
"Detection of Soluble Class I Molecules (non
HLA-A or HLA-B) in Serum Spleen Membranes
and Lympocytes in Culture", pages 1835-1839,
see entire document.
• Curr. Opinion in Immunology, Volume 2, issued
1989, ROTHBARD, "Major Histocompatibility
Complex Peptide Interactions", pages 99-105.
• Immunol. Reviews, No. 81, issued 1984, KLEIN,
"What Causes Immunological Non
Responsiveness?", pages 177-202, see entire
article.
• Current Opinion in Immunology, Volume 2,
issued 1989, J.B. ROTHBARD, "Antigen
Recognition", pages 79-80, see entire document.
• Current Opinion in Immunology, Volume 2,
issued 1989, V.C. SCHAD et al., "T Cell
Accessory Molecules Mediating Cell Adhesion
and Signal Transduction", pages 123-128, see
entire article.

EP 0 630 255 B1

**Description**

BACKGROUND OF THE INVENTION

The invention relates to the methods and compositions for the modulation of T cell function in the treatment of for example, autoimmune diseases, allergic responses, transplant rejection, and other immunological disorders. In particular, it concerns complexes which target helper T cells by using a complex of the major histocompatibility complex (MHC) glycoproteins with peptides representing fragments of antigens associated with such diseases. These complexes can be further conjugated to radioisotopes or other labels for diagnostic purposes, or to toxins or other substances which render the complexes therapeutically useful.

A number of pathological responses involving unwanted T cell activation are known. For instance, a number of allergic diseases, have been associated with particular MHC alleles or suspected of having an autoimmune component.

Other deleterious T cell-mediated responses include the destruction of foreign cells that are purposely introduced into the body as grafts or transplants from allogeneic hosts. This process, known as "allograft rejection," involves the interaction of host T cells with foreign MHC molecules. Quite often, a broad range of MHC alleles are involved in the response of the host to an allograft.

Autoimmune disease is a particularly important class of deleterious immune response. In autoimmune diseases, self-tolerance is lost and the immune system attacks "self" tissue as if it were a foreign target. More than 30 autoimmune diseases are presently known; these include many which have received much public attention, including myasthenia gravis (MG) and multiple sclerosis (MS).

A crude approach to treating autoimmune disease and other immunopathologies is general immunosuppression. This has the obvious disadvantage of crippling the ability of the subject to respond to real foreign materials to which it needs to mount an immune response. An only slightly more sophisticated approach relies on the removal of antibodies or immune complexes involving the target tissue. This also has adverse side effects, and is difficult to accomplish. The invention approach, described in detail below, relies on a "clonotypic" reagent--i.e., a reagent which attacks only the cells of the immune system which are responsive to the autoantigen.

In the general paradigm now considered to describe the immune response, specific antigens presented result in a clonal expansion, as first proposed by Burnet in 1959. According to this scenario, a particular subject will have hundreds of thousands of T and B cells each bearing receptors that bind to different antigenic determinants. Upon exposure to an antigen, the antigen selectively binds to cells bearing the appropriate receptors for the antigenic determinants it contains, ignoring the others. The binding results in a cloned population of thousands of daughter cells, each of which is marked by the same receptor. A clonotypic reagent affects only a subset of the T and B cells which are appropriate for the antigen of interest. In the case of the invention compositions, the antigenic determinant is usually that associated with an autoimmune disease.

The clonotypic reagent compositions of the invention are specifically designed to target T-helper cells which represent the clones specific for the antigenic determinant(s) of the tissue which is affected by the autoimmune disease. T-helper cells recognize a determinant only in association with an MHC protein; the complexes of the invention therefore include an effective portion of the MHC protein.

There have, recently, been some related approaches which attempt to interdict the immune response to specific antigens. For example, the autoantigen thyroglobulin has been conjugated to ricin A and the conjugate was shown to suppress specifically the in vitro antibody response of lymphocytes which normally respond to this antigen. It was suggested that such immunotoxins would specifically delete autoantibody-secreting lymphocyte clones (Rennie, D.P., et al., Lancet (Dec. 10, 1983) 1338-1339). Diener, E., et.al., science (1986) 231:148-150 suggested the construction of compounds which cause antigen-specific suppression of lymphocyte function by conjugating daunomycin to the hapten (in this case, of ovalbumin) using an acid-sensitive spacer. The conjugate caused hapten-specific inhibition of antibody secretion by B lymphocytes in vitro and in vivo. A conjugate of daunomycin (with an acid-sensitive spacer) to a monoclonal antibody-specific to T cells also eliminated the response by T-lymphocytes to concanavalin A. Steerz, R.K.M., et al., J. Immunol. (1985) 134:841-846 utilized radiation as the toxic element in a toxin conjugate. Rats were administered a radioactively labeled, purified receptor from electric fish, prior to injection with cold receptor. Injection with this receptor is a standard procedure to induce experimental autoimmune myasthenia gravis (EAMG). Control rats that received preinjection only either of cold receptor or radiolabeled albumin, prior to administration of receptor to induce the disease develop the symptoms of EAMG; those pretreated with radioactively-labeled receptor showed reduced symptoms. It was surmised that the labeled, and therefore destructive, receptor selectively eliminated immunocompetent cells. Similar work utilizing a ricin/receptor conjugate for pretreatment was reported by Killen, J.A., et al., J. Immunol. (1984) 133:2549-2553.

A less specific approach which results in the destruction of T cells in general is treatment with an IL-2/toxin conjugate as reported by Hixson, J.R., Medical Tribune (Jan. 28, 1988) 4-5. In a converse, but related, approach Liu, M.A., et al., Science (1988) 239:395-397, report a method to "link up" cytotoxic T cells with a desired target, regardless of the cytotoxic T cell specificity. In this approach, antibody specific to the universal cytotoxic T-lymphocytes to destroy human melanoma cells when melanocyte-stimulating hormone was the hormone used.

The current model of immunity postulates that antigens mobilize an immune response, at least in part, by being ingested by an antigen-presenting cell (APC) which contains on its surface a Class II glycoprotein encoded by a gene in the major histocompatibility complex (MHC). The antigen is then presented to a specific T helper cell in the context of the surface bound MHC glycoprotein, and by interaction of the antigen specific T cell receptor with the antigen -MHC complex, the T helper cell is stimulated to mediate the antigen-specific immune response, including induction of cytotoxic T cell function, induction of B cell function, and secretion of a number of factors aiding and abetting this response.

The involvement of the MHC Class II proteins in autoimmune disease has been shown in animal models. Administration of antibodies to either MHC Class II proteins themselves or antibodies to agents that induce expression of the MHC Class II genes interferes with development of the autoimmune condition in these model systems. The role of helper T cells has also been demonstrated in these models by counteracting the autoimmune system using anti-CD4 monoclonal antibodies; CD4 is the characteristic helper T cell receptor (Shizuru, J.A. et al., Science (1988) 240: 659-662).

Recent experiments have shown that, under certain circumstances, anergy or nonresponsiveness can be induced in autoreactive lymphocytes (see, Schwartz, Cell (1989) 1073-1081). In vitro experiments suggest that antigen presentation by MHC Class II molecules in the absence of a co-stimulatory signal induces a state of proliferative non-responsiveness in syngeneic T cells (Quill et al., J. Immunol. (1987) 138:3704-3712). These reports, however, provide no clear evidence that induction of anergy in vivo is possible or that autoimmune disease can be effectively treated in this manner.


SUMMARY OF THE INVENTION

The present invention provides a substantially pure MHC-peptide complex consisting of an antigenic peptide consisting of residues 84-102 of human MBP or a segment of 8 to 18 amino acids thereof, and an isolated MHC component having an antigen binding site, wherein the antigenic peptide is associated with the antigen binding site.

The invention further provides a pharmaceutical composition comprising a pharmaceutically acceptable carrier and said complex.

The invention further provides a method for preparing a complex of the invention, the method comprising: isolating the MHC component from a cell producing the component; contacting the MHC component with the peptide such that the peptide is coupled to the antigen binding site; and removing excess peptide not coupled to the antigen binding site.

These and other aspects of the invention are described herein and in the appended claims.

The invention compositions and methods are designed to target helper T cells which recognise a particular antigen in association with a glycoprotein encoded by the MHC. The invention complexes bind T cell receptors and cause non-responsiveness in target T-lymphocytes and other cells of the immune system.

Compositions of the present invention are purified two component complexes. These two components may be bound covalently or by noncovalent association. Evidence from both in vitro or in vivo experiments establishes that such complexes induce clonal anergy in syngeneic T cells.

Compositions of the invention can be used to down-regulate parts of the immune system reactive with a particular antigen associated with an immune response, such as autoimmune disease.


BRIEF DESCRIPTION OF THE DRAWINGS

Fig. 1 is a diagram of a typical complex of the invention.
Fig. 2 shows the 3-dimensional structure of the human HLA-A2 antigen (Class I).
Fig. 3 shows a diagrammatic representation of the active portion of a modified Class II MHC-encoded glycoprotein.
Fig. 4 shows preferred second generation MHC protein designs.
Fig. 5 is a diagram of a planar membrane bilayer including the MHC glycoprotein, mimicking the surface of the antigen presenting cell.
Fig. 6 shows the amino acid sequence and encoding MRNA for the alpha subunit of acetylcholine receptor protein.
Fig. 7 shows the amino acid sequence of myelin basic protein.
Fig. 8 shows the nucleotide sequence encoding the I-A$^b$-alpha chain.
Fig. 9 shows the nucleotide sequence encoding the I-A$^b$-beta chain.

Fig. 10 presents a list of the DQ/DR haplotypes in humans and their associations with autoimmune diseases.

Fig. 11 shows a protocol suitable for the utilization of the complexes of the invention for the diagnosis and/or treatment of an autoimmune disease.

Fig. 12 shows a scheme for the preparation of I-A$^k$ containing NP-40 soluble membrane extracts.

Fig. 13A is a scheme for the affinity purification of 10-2.16 monoclonal antibody and its coupling to CNBr activated Sepharose 4B.

Fig. 13B is a copy of a gel showing the purity of 10-2.16 monoclonal antibody purified by the scheme in Fig. 13A.

Fig. 14 shows a scheme for the purification of I-A$^k$.

Fig. 15 is a polyacrylamide gel showing the purity of I-A$^k$ purified by the scheme in Fig. 14.

Fig. 16 is a bar graph showing the results of eight studies on the inhibition of proliferation by a complex containing I-A$^k$ and MBP (1-13).

Fig. 17 is a graph showing the development of EAE in mice resulting from immunization with MBP (1-13).

Fig. 18 is a graph showing the adoptive transfer of EAE by T cell clone 4R3.4, obtained from B10A(4R) strain of mice following immunization with MBP(1-11).

Figs. 19A-C show treatment of passively induced EAE in mice using PBS alone (19a), I-A$^s$/MBP1-14 (a non-encephalitogenic peptide) (19b), and I-A$^s$/MBp91-103 (19c).

Fig. 20 shows that complexes of the invention exist as aggregates because they pass through the column with the void volume and thus have a molecular weight greater than 600,000.

## DESCRIPTION OF PREFERRED EMBODIMENTS

The present invention provides complexes which can be used to modulate T cell function. For instance, the complexes can be used to inhibit a deleterious T cell-mediated immune response, such as allergic responses, allograft rejection, and autoimmune diseases. In addition, the complexes of the invention can be used to promote immune responses and be used as vaccines. In this emodiment, the MHC component (either Class I or Class II) is typically modified to allow attachment to a competent antigen presenting cell bearing ligands involved in the costimulatory signal. Alternatively, the complex may be linked to isolated costimulatory ligands such that T cell proliferation is induced. Thus, T cells will respond to the antigenic peptide presented by the complexes and an immune repsonse will be initiated.

The invention complexes contain at least two components: a peptide which represents an autoantigen or other antigenic sequence with the relevant effect on the immune system and an effective portion of the MHC-encoded glycoprotein involved in antigen presentation. An effective portion of an MHC glycoprotein is one which comprises the antigen binding sites and sequences necessary for recognition of the MHC-peptide complex by the appropriate T cell receptor. The MHC component can be either a Class I or a CLass II molecule. The association between the peptide antigen and the antigen binding sites of the MHC protein can be by covalent or by noncovalent bonding.

In other embodiments the complexes may also contain an effector component which is generally a toxin or a label. The effector portion may be conjugated to either the MHC-encoded glycoprotein or to the autoantigenic peptide. Complexes containing an effector component are disclosed and claimed in copending application U.S.S.N. 07/367,751 filed June 21, 1989, (US Patent 5,194,425).

Each of the components of the system is described separately below; followed by description of the methods by which these complexes can be prepared, evaluated and employed.

### The MHC-Derived Component

The glycoproteins encoded by the MHC have been extensively studied in both the human and murine systems. In general, they have been classified as Class I glycoproteins, found on the surfaces of all cells and primarily recognized by cytotoxic T cells; and Class II which are found on the surfaces of several cells, including accessory cells such as macrophages, and are involved in presentation of antigens to helper T cells. Some of the histocompatibility proteins have been isolated and characterized. For a general review of MHC glycoprotein structure and function, see Fundamental Immunology, 2d Ed., W.E. Paul, ed., Ravens Press N.Y. 1989. The term "isolated MHC component" as used herein refers to an MHC glycoprotein or an effective portion of an MHC glycoprotein (i.e., one comprising an antigen binding site or sites and sequences necessary for recognition by the appropriate T cell receptor) which is in other than its native state, for example, not associated with the cell membrane of a cell that normally expresses MHC. As described in detail below, the MHC component may be recombinantly produced, solubilized from the appropriate cell source or associated with a liposome. For human MHC molecules, human lymphoblastoisd cells are particularly preferred.

Methods for purifying the murine I-A (Class II) histocompatibility proteins have been disclosed by Turkewitz, A.P., et al., Molecular Immunology (1983) 20:1139-1147. These methods, which are also suitable for Class I molecules, involve preparation of a soluble membrane extract from cells containing the desired MHC molecule using nonionic detergents, such as NP-40, Tween 80 and the like. The MHC molecules are then purified by affinity chromatography, using a column containing antibodies raised against the desired MHC molecule. Use of 0.02% Tween-80 in the elution buffer is helpful to eliminate aggregation of the purified molecules.

The isolated antigens encoded by the I-A and I-E subregions have been shown to consist of two noncovalently bonded peptide chains: an alpha chain of 32-38 kd and a beta chain of 26-29 kd. A third, invariant, 31 kd peptide is noncovalently associated with these two peptides, but it is not polymorphic and does not appear to be a component of the antigens on the cell surface (Sekaly, R.P., J. Exp. Med. (1986) 164:1490-1504. The alpha and beta chains of seven allelic variants of the I-A region have been cloned and sequenced (Estees, "T cell Clones", 3-19).

The human Class I proteins have also been studied. The MHC of humans (HLA) on chromosome 6 has three loci, HLA-, HLA-B, and HLA-C, the first two of which have a large number of alleles encoding alloantigens. These are found to consist of a 44 kd subunit and a 12 kd beta$_2$-microglobulin subunit which is common to all antigenic specificities. Isolation of these detergent-soluble HLA antigens was described by Springer, T.A., et al., Proc. Natl. Acad. Sci. USA (1976) 73:2481-2485; Clementson, K.J., et al., in "Membrane Proteins" Azzi, A., ed; Bjorkman, P., Ph.D. Thesis Harvard (1984).

Further work has resulted in a detailed picture of the 3-D structure of HLA-A2, a Class I human antigen. (Bjorkman, P.J., et al., Nature (1987) 329:506-512, 512-518. In this picture, the $\beta_2$-microglobulin protein and alpha$_3$ segment of the heavy chain are associated; the alpha$_1$ and alpha$_2$ regions of the heavy chain appear to form antigen-binding sites to which the peptide is bound (Science (1987) 238:613-614. Bjorkman, P.J. et al. Nature (supra). Soluble HLA-A2 can be purified after papain digestion of plasma membranes from the homozygous human lymphoblastoid cell line J-Y as described by Turner, M.J. et al., J. Biol. Chem. (1977) 252:7555-7567. Papain cleaves the 44 kd chain close to the transmembrane region yielding a molecule comprised of alpha$_1$, alpha$_2$, alpha$_3$, and $\beta_2$ microglobulin. A representation of the deduced three dimensional structure of the Class I HLA-A2 antigen is shown in Fig. 2.

While the three dimensional structure of Class II MHC antigens is not known in such detail, it is thought that Class II glycoproteins have a domain structure, including an antigen binding site, similar to that of Class I. It is formed from the N-terminal domain portions of two class II chains which extend from the membrane bilayer. The N-terminal portion of one chain has two domains of homology with the alpha$_1$ and alpha$_2$ regions of the MHC Class I antigen sequence. Cloning of the Class II genes (as described by Estees supra) permits manipulation of the Class II MHC binding domains for example, as described below.

The MHC glycoprotein portions of the complexes of the invention, then, can be obtained by isolation from lymphocytes and screened for the ability to bind the desired peptide antigen. The lymphocytes are from the species of individual which will be treated with the complexes. For example, they may be isolated from human B cells from an individual suffering from the targeted autoimmune disease, which have been immortalized by transformation with a replication deficient Epstein-Barr virus, utilizing techniques known in the art.

MHC glycoproteins have been isolated from a multiplicity of cells using a variety of techniques including solubilization by treatment with papain, by treatment with 3M KCl, and by treatment with detergent. In a preferred method detergent extraction of Class II protein from lymphocytes followed by affinity purification is used. Detergent can then be removed by dialysis or selective binding beads, e.g., Bio Beads.

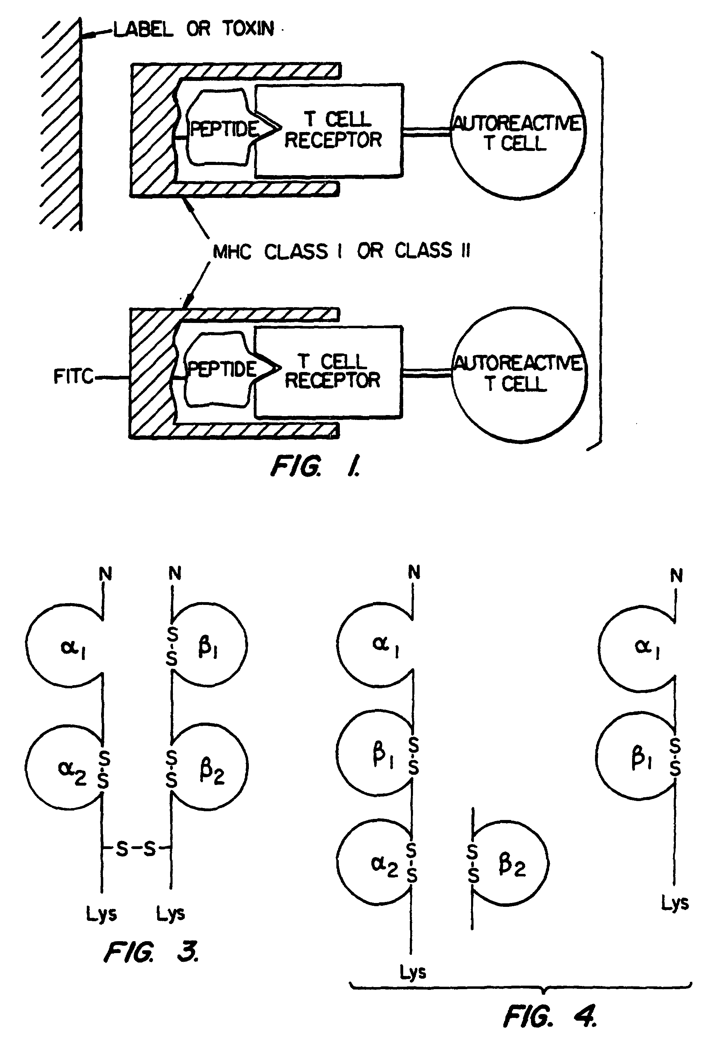

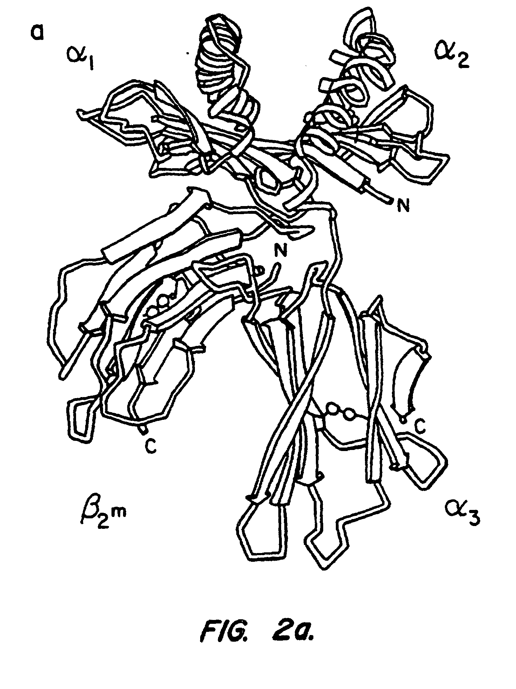

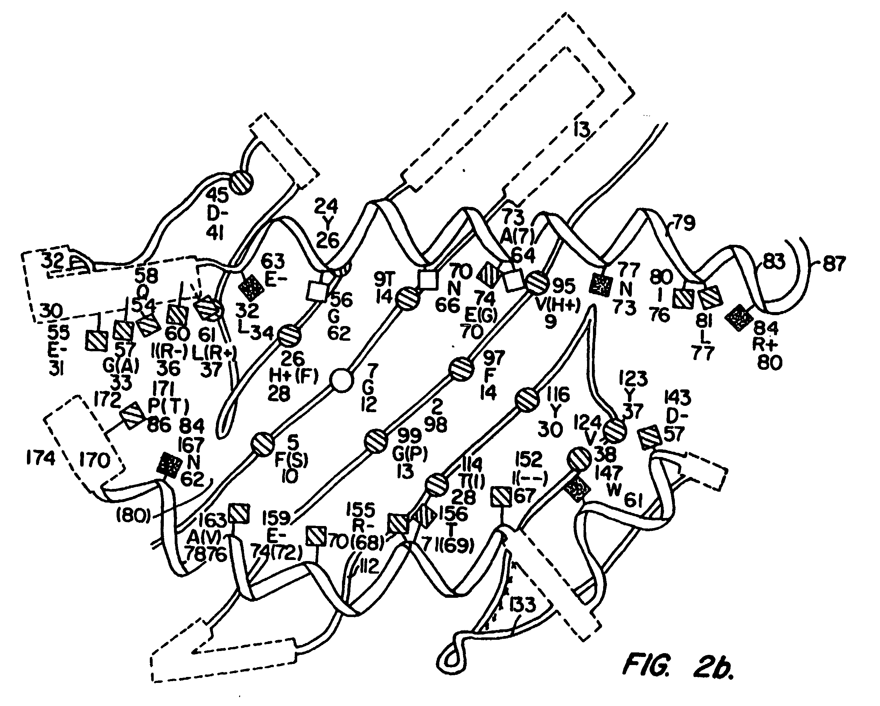

Alternatively, the amino acid sequence of each of a number of Class II proteins are known, and the genes have been cloned, therefore, the proteins can be made using recombinant methods. In a first generation synthetic MHC protein, the heavy (alpha) and light (beta) chains are synthesized using a carboxy terminal truncation which effects the deletion of the hydrophobic domain, and the carboxy termini can be arbitrarily chosen to facilitate the conjugation of toxins or label. For example, in the MHC protein shown in Figure 3, lysine residues are introduced. In addition, cysteine residues near the carboxy termini are included to provide a means to form disulfide linkage of the chains; the synthetic gene can also include restriction sites to aid in insertion into expression vectors and in manipulating the gene sequence to encode analogs. The alpha and beta chains are then inserted into expression vectors, expressed separately in an appropriate host, such as E. coli, yeast, or other suitable cells, and the recombinant proteins obtained are recombined in the presence of the peptide antigen.

As the availability of the gene permits ready manipulation of the sequence, a second generation of preferred construction includes hybrid Class I and Class II features, as illustrated in Figure 4, wherein the alpha$_1$ and beta$_1$ domains of Class II MHC are linked through a flexible portion that permits intramolecular dimerization between these domains resulting in an edge-to-edge beta sheet contact. The beta$_1$ segment is then fused to the alpha$_2$ domain of Class I with beta$_2$ microglobulin coexpressed to stabilize the complex. The transmembrane and intracellular domains of the Class I gene can also be included but there may be no point in doing so unless liposomes are used to transport the complex. A simpler version includes only the alpha$_1$ and beta$_1$ domains with a C-terminal lysine for toxin conjugation (Figure 4).

Construction of expression vectors and recombinant production from the appropriate DNA sequences are performed by methods known in the art per se. Expression can be in procaryotic or eucaryotic systems. Procaryotes most frequently are represented by various strains of E. coli. However, other microbial strains may also be used, such as bacilli, for example Bacillus subtilis, various species of Pseudomonas, or other bacterial strains. In such procaryotic systems, plasmid vectors which contain replication sites and control sequences derived from a species compatible with the host are used. For example, E. coli is typically transformed using derivatives of pBR322, a plasmid derived from an E. coli species by Bolivar et al., Gene (1977) 2:95. Commonly used procaryotic control sequences, which are defined herein to include promoters for transcription initiation, optionally with an operator, along with ribosome binding site sequences, including such commonly used promoters as the beta-lactamase (penicillinase) and lactose (lac) promoter systems (Change et al., Nature (1977) 198:1056) and the tryptophan (trp) promoter system (Goeddel et al., Nucleic Acids Res. (1980) 8:4057) and the lambda-derived P_L promoter and N-gene ribosome binding site (Shimatake et al., Nature (1981) 292:128). Any available promoter system compatible with procaryotes can be used.

The expression systems useful in the eucaryotic hosts comprise promoters derived from appropriate eucaryotic genes. A class of promoters useful in yeast, for example, include promoters for synthesis of glycolytic enzymes, including those for 3-phosphoglycerate kinase (Hitzeman, et al., J. Biol. Chem. (1980) 255:2073). Other promoters include those from the enolase gene (Holland, M.J., et al. J. Biol. Chem. (1981) 256:1385) or the Leu2 gene obtained from YEp13 (Broach, J., et al., Gene (1978) 8:121).

Suitable mammalian promoters include the early and late promoters from SV40 (Fiers, et al., Nature (1978) 273:113) or other viral promoters such as those derived from polyoma, adenovirus II, bovine papilloma virus or avian sarcoma viruses. Suitable viral and mammalian enhancers are cited above.

The expression system is constructed from the foregoing control elements operably linked to the MHC sequences using standard methods, employing standard ligation and restriction techniques which are well understood in the art. Isolated plasmids, DNA sequences, or synthesized oligonucleotides are cleaved, tailored, and relegated in the form desired.

Site specific DNA cleavage is performed by treating with the suitable restriction enzyme (or enzymes) under conditions which are generally understood in the art, and the particulars of which are specified by the manufacturer or these commercially available restriction enzymes. See, e.g., New England Biolabs, Product Catalog. In general, about 1 ug of plasmid or DNA sequence is cleaved by one unit of enzyme in about 20 ul of buffer solution; in the examples herein, typically, an excess of restriction enzyme is used to insure complete digestion of the DNA substrate. Incubation times of about 1 hr to 2 hr at about 37°C are workable, although variations can be tolerated. After each incubation, protein is removed by extraction with phenol/chloroform, and may be followed by ether extraction, and the nucleic acid recovered from aqueous fractions by precipitation with ethanol followed by running over a Sephadex G-50 spin column. If desired, size separation of the cleaved fragments may be performed by polyacrylamide gel or agarose gel electrophoresis using standard techniques. A general description of size separation is found in Methods in Enzymology (1980) 65:499-560.

Restriction cleaved fragments may be blunt ended by treating with the large fragment of E. coli DNA polymerase I (Klenow) in the presence of the four deoxynucleotide triphosphates (dNTPs) using incubation times of about 15 to 25 min at 20 to 25°C in 50 Mm Tris Ph 7.6, 50 Mm NaCl, 6 mM MgCl$_2$, 6 Mm DTT and 5-10 uM dNTPs. The Klenow fragment fills in a 5' sticky ends but chews back protruding 3' single strands, even through the four dNTPS, are present. If desired, selective repair can be performed by supplying only one of the, or selected, dNTPs within the limitations dictated by the nature of the sticky ends. After treatment with Klenow, the mixture is extracted with phenol/chloroform and ethanol precipitated followed by running over a Sephadex G-50 spin column.

synthetic oligonucleotides are prepared using commercially available automated oligonucleotide synthesizers. Kinasing of single strands prior to annealing or for labeling is achieved using an excess, e.g., approximately 10 units of polynucleotide kinase to 0.1 nmole substrate in the presence of 50 mM Tris, pH 7.6, 10 mM MgCl$_2$, 5 mM dithiothreitol, 1-2 mM ATP, 1.7 pmoles $^{32}$P-ATP (2.9 mCi/mmole), 0.1 mM spermidine, 0.1 mM EDTA.

Ligations are performed in 15-30 ul volumes under the following standard conditions and temperatures: 20 mM Tris-HCl pH 7.5, 10 mM MgCl$_2$, 10 mM DTT, 33 ug/ml BSA, 10 mM-50 mM NaCl, and either 40 uM ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 33-100 ug/ml total DNA concentrations (5-100 nM total end concentration). Intermolecular blunt end ligations (usually employing a 10-30 fold molar excess of linkers) are performed at 1 uM total ends concentration.

In vector construction employing "vector fragments," the vector fragment is commonly treated with bacterial alkaline phosphatase (BAP) in order to remove the 5' phosphate and prevent religation of the vector. BAP digestions are conducted at pH 8 in approximately 150 mM Tris, in the presence of Na$^+$ and Mg$^{+2}$ using about 1 unit of BAP per ug of vector at 60°C for about 1 hr. In order to recover the nucleic acid fragments, the preparation is extracted with phenol/chloroform and ethanol precipitated and desalted by application to a Sephadex G-50 spin column. Alternatively, religation can be prevented in vectors which have been double digested by additional restriction enzyme digestion of the unwanted fragments.

For portions of vectors derived from cDNA or genomic DNA which require sequence modifications, site specific primer directed mutagenesis can be used. This is conducted using a primer synthetic oligonucleotide complementary to a single stranded phage DNA to be mutagenized except for limited mismatching, representing the desired mutation.

Briefly, the synthetic oligonucleotide is used as a primer to direct synthesis of a stand complementary to the phage, and the resulting double-stranded DNA is transformed into a phage-supporting host bacterium. Cultures of the transformed bacteria are plated in top agar, permitting plaque formation from single cells which harbor the phage.

Theoretically, 50% of the new plaques will contain the phage having, as a single strand, the mutated form; 50% will have the original sequence. The resulting plaques are hybridized with kinased synthetic primer at a temperature which permits hybridization of an exact match, but at which the mismatches with the original strand are sufficient to prevent hybridization. Plaques which hybridize with the probe are then picked, cultured, and the DNA recovered.

In the proteins of the invention, however, a synthetic gene is conveniently employed. The gene design can include restriction sites which permit easy manipulation of the gene to replace coding sequence portions with these encoding analogs.

Correct ligations for plasmid construction can be confirmed by first transforming E. coli strain MM294 obtained from E. coli Genetic Stock Center, CGSC #6135, or other suitable host with the ligation mixture. Successful transformants are selected by ampicillin, tetracycline or other antibiotic resistance or using other markers depending on the-mode of plasmid construction, as is understood in the art. Plasmid from the transformants are then prepared according to the method of Clewell, D.B., et al., Proc. Natl. Acad. Sci. USA (1969) 62:1159, optionally following chloramphenicol amplification (Clewell, D.B., J. Bacteriol. (1972) 110:667). The isolated DNA is analyzed by restriction and/or sequenced by the dideoxy method of Sanger, F., et al., Proc. Natl. Acad. Sci. USA (1977) 74:5463 as further described by Messing, et al., Nucleic Acids Res. (1981) 9:309, or by the method of Maxam, et al., Methods in Enzymology (1980) 65:499.

The constructed vector is then transformed into a suitable host for production of the protein. Depending on the host cell used, transformation is done using standard techniques appropriate to such cells. The calcium treatment employing calcium chloride, as described by Cohen, S.N., Proc. Natl. Acad. Sci. USA (1972) 69:2110, or the RbC1 method described in Maniatis, et al., Molecular Cloning: A Laboratory Manual (1982) Cold Spring Harbor Press, p. 254 is used for procaryotes or other cells which contain substantial cell wall barriers. For mammalian cells without such cell walls, the calcium phosphate precipitation method of Graham and van der Eb, Virology (1978) 52:546 or electroporation is preferred. Transformations into yeast are carried out according to the method of Van Solingen, P., et al., J. Bacter. (1977) 130:946 and Hsiao, C.L., et al., Proc. Natl. Acad. Sci. USA (1979) 76:3829.

The transformed cells are then cultured under conditions favoring expression of the MHC sequence and the recombinantly produced protein recovered from the culture.

## Antigenic Peptides

The autoantigenic proteins or tissues for a number of autoimmune diseases are known. For example, in experimentally induced autoimmune diseases, antigens involved in pathogenesis have been characterized: in arthritis in rat and mouse, native type-II collagen is identified in collagen-induced arthritis, and mycobacterial heat shock protein in adjuvant arthritis (Stuart et al. (1984), Ann. Rev. Immunol. 2:199-218; van Eden et al. (1988), Nature 331:171-173.); thyroglobulin has been identified in experimental allergic thyroiditis (EAT) in mouse (Maron et al. (1988), J. Exp. Med. 152:1115-1120); acetyl choline receptor (AChR) in experimental allergic myasthenia gravis (EAMG) (Lindstrom et al. (1988), Adv. Immunol. 42:233-284); and myelin basic protein (MBP) and proteolipid protein (PLP) in experimental allergic encephalomyelitis (EAE) in mouse and rat (See Acha-Orbea et al., supra). In addition, for example, target antigens have been identified in humans: type-II collagen in human rheumatoid arthritis (Holoshitz et al. (1986), Lancet ii:305-309); and acetyl choline receptor in myasthenia gravis (Lindstrom et al. (1988), supra).

It is believed that the presentation of antigen by the MHC glycoprotein on the surface of antigen-presenting cells (APCs) occurs subsequent to the hydrolysis of antigenic proteins into smaller peptide units. The location of these smaller segments within the antigenic protein can be determined empirically. These segments are thought to be 8-18 residues in length, and contain both the agretope (recognized by the MHC molecule) and the epitope (recognized by T cell receptor on the T-helper cell). The epitope itself is a contiguous or non-contiguous sequence of 5-6 amino acids which recognizes the antigen-specific receptor of T-helper cells. The agretope is a continuous or non-contiguous sequence which is responsible for the association of the peptide with the MHC glycoproteins.

The empirical process of determining the relevant 8-18 amino acid subunits is illustrated using the alpha subunit of the acetylcholine receptor of skeletal muscle. In myasthenia gravis (MG) an autoimmune response is directed to a region of this subunit. A loss of the acetyl choline receptors on the postsynaptic membrane of the neuromuscular junction causes the MG symptoms.

In MG, autoantibodies against the alpha subunit of the acetylcholine receptor (AChR) are associated with the autoimmune response directed at the AChR. Eighty five percent of MG patients have autoantibodies reactive with the alpha subunit. Of these, 60% have antibodies that bind to a peptide segment of the alpha subunit called the main immunogenic region (MIR) which is located between residues 60 and 80 (Tzartos and Lindstrom, Proc. Natl. Acad. Sci. USA (1980) 77:755). The peptide segments recognized by autoreactive human T cells also are located on the alpha subunit (Hohfield, et al., Proc. Natl. Acad. Sci. USA (1987). The epitopes recognized by these T cells lie between residues 1-30, 125-147, 169-181, 257-271 and 351-368. In addition, in humans the AChR peptides 195-212 and 257-269 have been partially characterized as epitopes in myasthenia gravis patients of the HLA-DR5 and HLA-DR3, DQw2 MHC haplotypes, respectively (See Acha-Orbea (1989), supra).

The peptides carrying agretopes permitting presentation of the epitopes associated with alpha subunit of this receptor are readily determined. For example, determination of the appropriate peptides in a mouse model is carried out as follows.

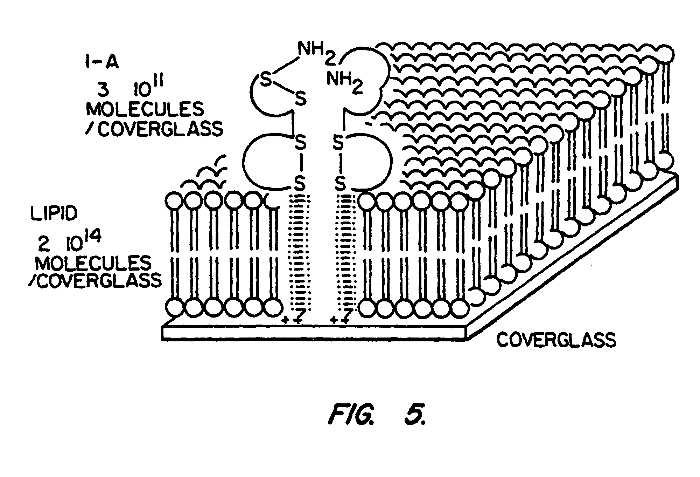

Strains of mice which, when immunized with Torpedo californicus AChR develop a disease with many of the features of human myasthenia gravis, are used as model. MHC Class II glycoproteins are isolated from spleen cells of mice of this strain using lectin and monoclonal antibody affinity supports. The purified MHC Class II proteins are incorporated into phospholipid vesicles by detergent dialysis. The resultant vesicles are then allowed to fuse to clean glass cover slips to produce on each a planar lipid bilayer containing MHC molecules as shown in Figure 5 (Brian and McConnell, Proc. Natl. Acad. Sci. USA (1984) 81:6159).

One cover slip containing MHC Class II molecules embedded in the adherent planar lipid membrane is placed in each well of several 24-well culture plates. Each one of the approximately 40 overlapping 20-residue synthetic peptides corresponding to the alpha subunit sequence and containing one or more radiolabeled amino acid residues (prepared as described below) is placed in a well with cover slip and PBS and allowed to incubate several days. The extent of binding of peptide in the MHC Class II glycoprotein antigen binding site is measured by the amount of radio-activity incorporated into the MHC Class II-planar lipid membrane on the cover slip versus planar lipid membrane alone. Specific incorporation of radioactivity indicates that the bound peptide contains an agretope (MHC Class II peptide binding site) of one of the several species of MHC Class II molecules present in the planar lipid membrane. In this way, the set of agretopes for the alpha subunit of AChR is defined for the mouse strain that displays the symptoms of MG upon immunization with AChR or purified alpha subunit.

Next, each of the alpha subunit synthetic peptide segments that contain an agretope is again incorporated into the antigen binding site of isolated MHC Class II proteins embedded in planar lipid membranes on cover slips. One cover slip is added to each well of a 24-well culture plate, and spleen cells from mice immunized against AChR (and from which strain the adherent MHC Class II proteins were isolated) are added to each well. T cell hybridoma proliferation, as measured by tritiated thymidine uptake into DNA, indicates that the MHC Class II protein-bound peptide contains both an agretope and an epitope for binding to the T cell. Activation of T cell clones is determined by measuring IL-3 production (see, Quill et al., supra).

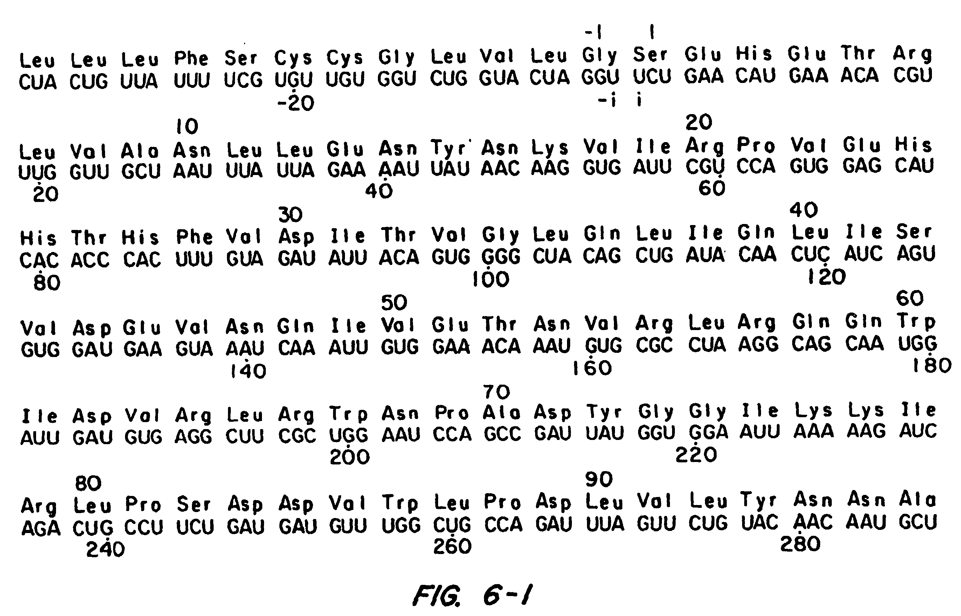

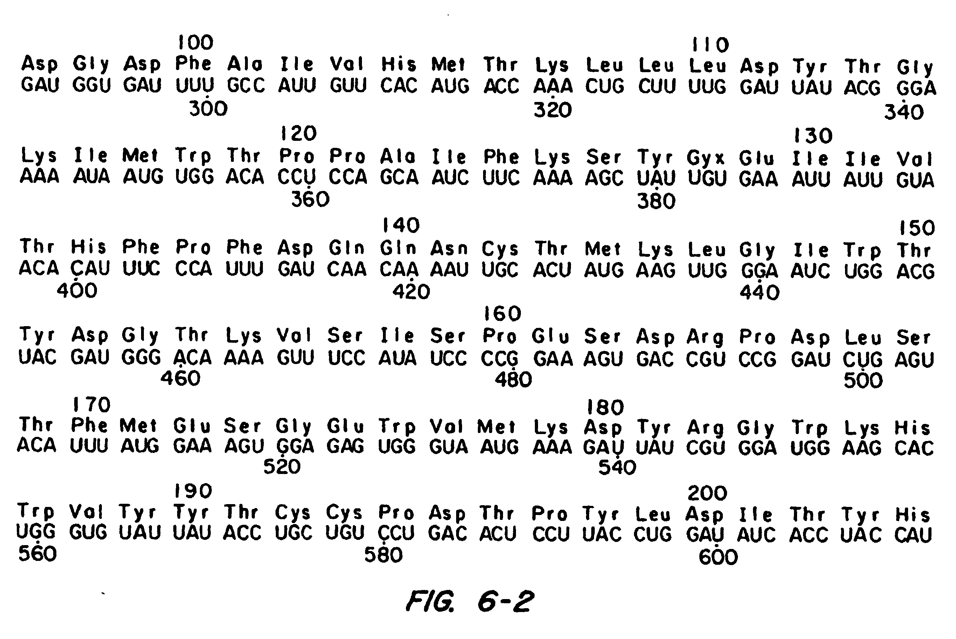

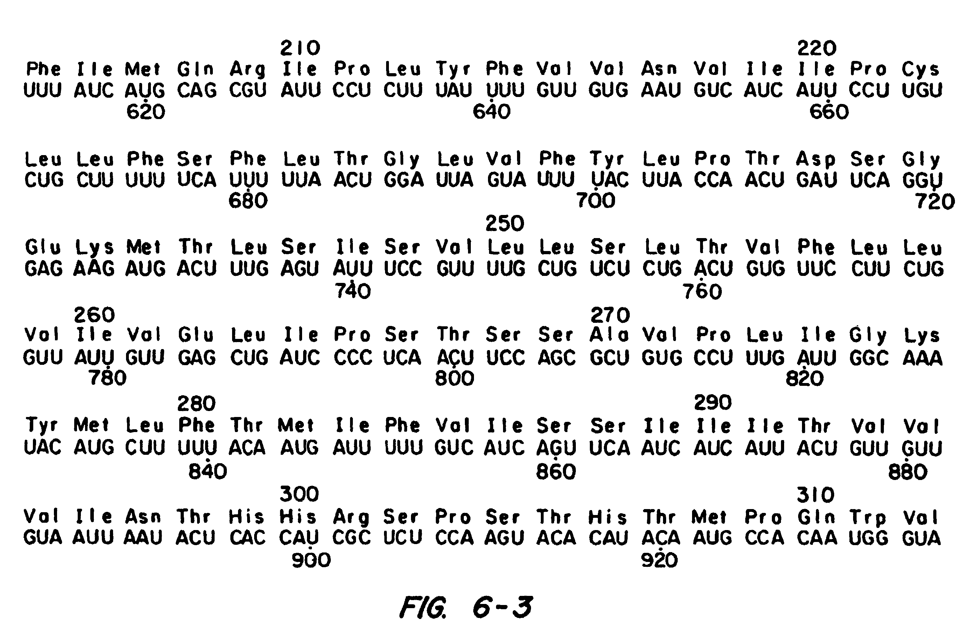

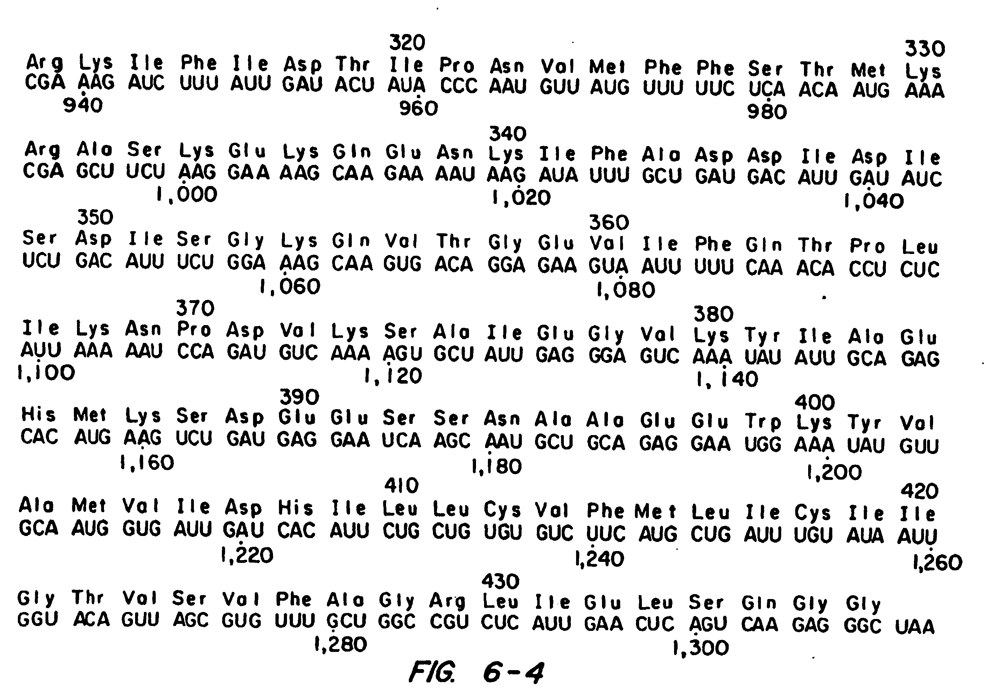

The Dupont apparatus and technique for rapid multiple peptide synthesis (RAMPS) is used to synthesize the members of a set of overlapping (10 residue overlap), 20-residue peptides from the alpha subunit of Torpedo californicus AChR. The sequence of this peptide is known and is shown in Figure 6. One or more radioactive amino acids is incorporated into each synthetic peptide. The pentafluorphenyl active esters of side chain-protected, FMOC amino acids are used to synthesize the peptides, applying standard stepwise solid phase peptide synthetic methods, followed by standard side chain deprotection and simultaneous release of the peptide amide from the solid support.

Alternatively the overlapping sequences which include the putative segments of 8-18 amino acids of the antigenic protein, such as acetylcholine receptor protein, can be synthesized on the method of Geysen, H.M., et al. J. Immun. Meth. (1987) 102:274. The synthesized radio labeled peptides are tested by incubating them individually (on the plates) with purified MHC proteins which have been formulated into lipid membrane bilayers as above.

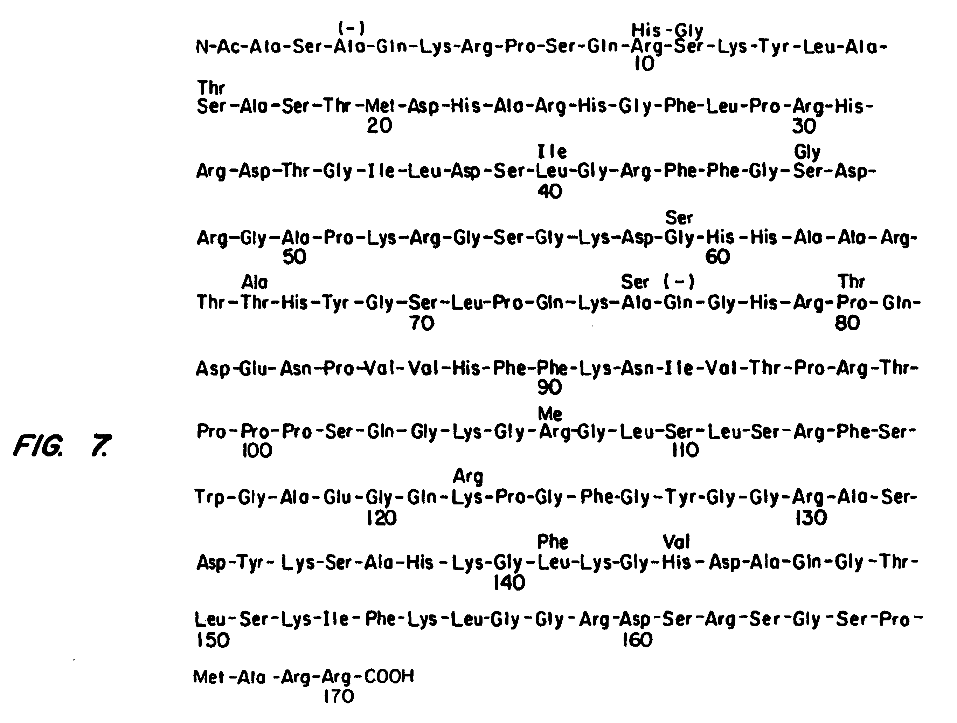

In multiple sclerosis (MS), which results in the destruction of the myelin sheath in the central nervous system, myelin basic protein (MBP), the major protein component of myelin is the principal autoantigen. Pertinent segments of the MBP protein are also determined empirically, using a strain of mice which develops experimental allergic encephalitis (EAG) when immunized with bovine myelin basic protein, the sequence of MBP is shown in Figure 7.

Systemic lupus erythematosus (SLE) has a complex systemology, but results from an autoimmune response to red blood cells. Peptides which are the antigenic effectors of this disease are found in the proteins on the surface of red blood cells.

Rheumatoid arthritis (RA) is a chronic inflammatory disease resulting from an immune response to proteins found in the synovial fluid.

Insulin-dependent diabetes mellitus (IDDM) results from autoimmune attack on the beta cells within the Islets of Langerhans which are responsible for secretion of insulin. Circulating antibodies to Islets cells surface antigens and to insulin are known to precede IDDM. Critical peptides in eliciting the immune response in IDDM are believed to be portions of the insulin sequence and the beta cell membrane surface proteins.

The relevant antigenic peptide subunits, as they are relatively short, can readily by synthesized using standard automated methods for peptide synthesis. In the alternative, they can be made recombinantly using isolated or synthetic DNA sequences; though this is not the most efficient approach for peptides of this length.

Thus, in summary, a set of labeled test peptides is prepared, and those which bind to MHC in planar lipid membranes containing MHC proteins are shown to contain the agretope.

The identified peptides are then prepared by conventional solid phase synthesis and the subset which contain epitopes for the disease-inducing helper T cell clones is determined by incubation of the candidate peptides with murine antigen-presenting cells (APC) (or with isolated MHC complex) and spleen or lymph node T cells from mice immunized with the full length protein. Successful candidates will stimulate T cell proliferation in this system. This second, smaller, subset represents the suitable peptide component.

Formation of the Complex

The elements of the complex can be associated by standard means known in the art. The antigenic peptides can be associated noncovalently with the pocket portion of the MHC protein by, for example, mixing the two components. Excess peptide can be removed any of a number of standard procedures, such as ultrafiltration or dialysis. The peptides can also be covalently bound using standard procedures by, for example, photo affinity labelling, (see e.g., Hall et al., Biochemistry 24:5702-5711 (1985)).

For example, the AChR peptide 195-215, which has been characterized as an epitope in MG in humans and in mice, may be connected to the N-terminal antigen binding site of a polypeptide derived from an MHC antigen associated with MG. The amino acid sequence of the AChR peptide in one letter amino acid code is:


**DTPYLDITYHFIMQRIPLYFV**


An oligonucleotide which encodes the peptide is synthesized using the known codons for the amino acid, preferably those codons which have preferred utilization in the organism which is to be used for expression are utilized in designing the oligonucleotide. Preferred codon utilizations for a variety of organisms and types of cells are known in the art. If, for example, expression is to be in E. coli, a suitable oligonucleotide sequence encoding AChR 195-215 could be:


**5′ GAC ACC CCG TAC CTG GAC ATC ACC TAC CAC TTC ATC
ATG CAG CGT ATC CCG CTG TAC TTC CTG 3′.**


This sequence may then be incorporated into a sequence encoding the peptides derived from the MHC antigen, utilizing techniques known in the art. The incorporation site will be such that, when the molecule is expressed and folded, the AChR peptide antigen will be available as an epitope for the target T cells.

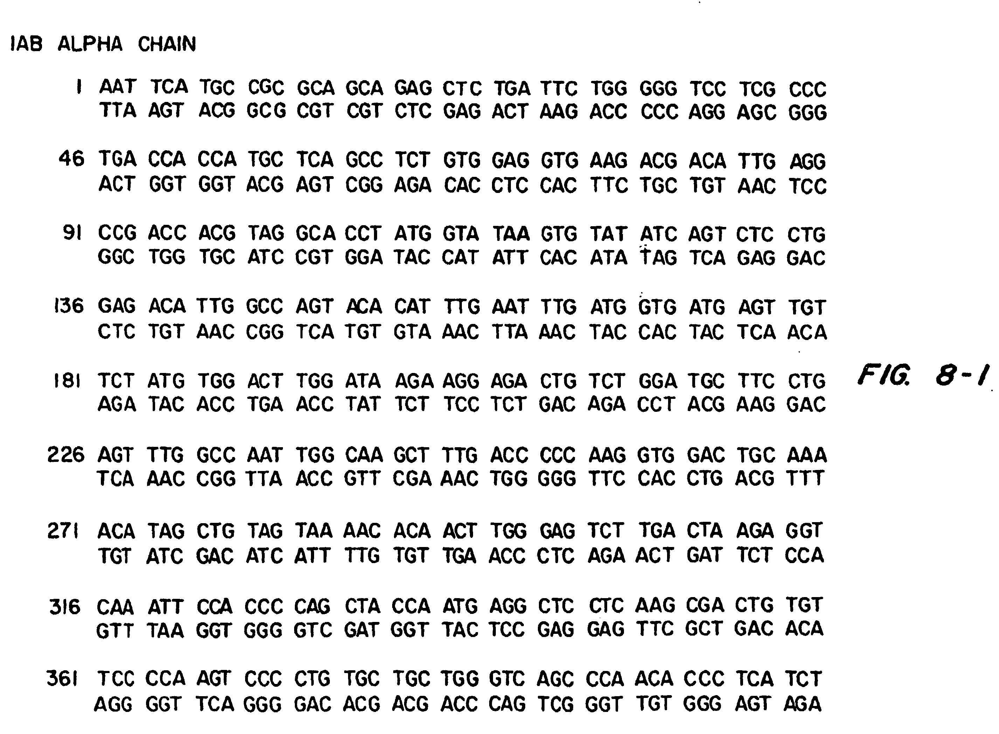

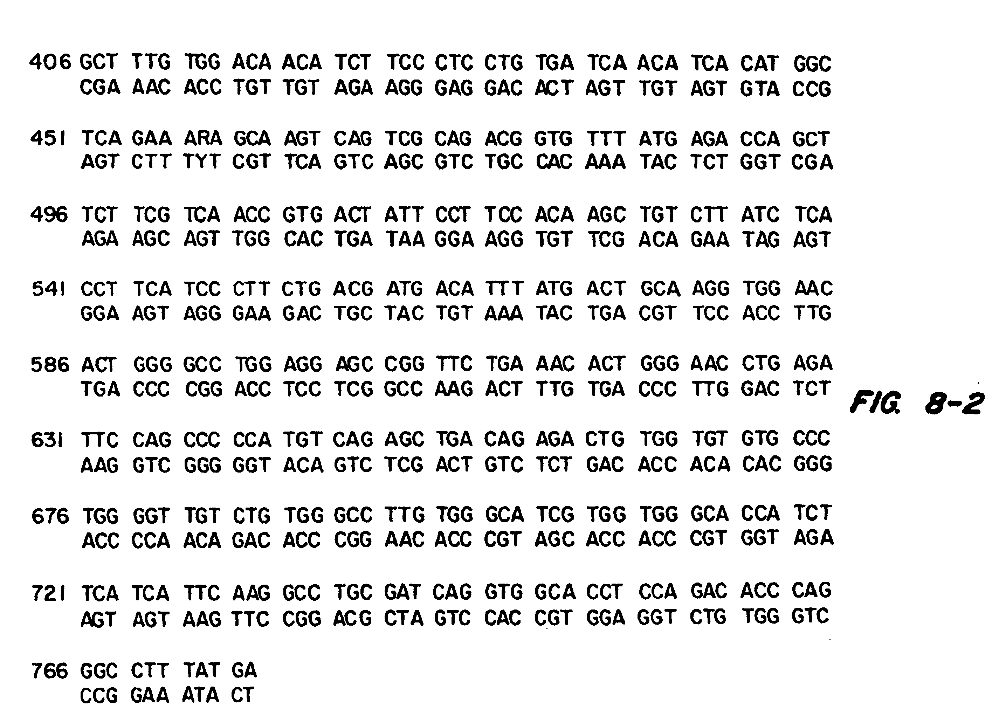

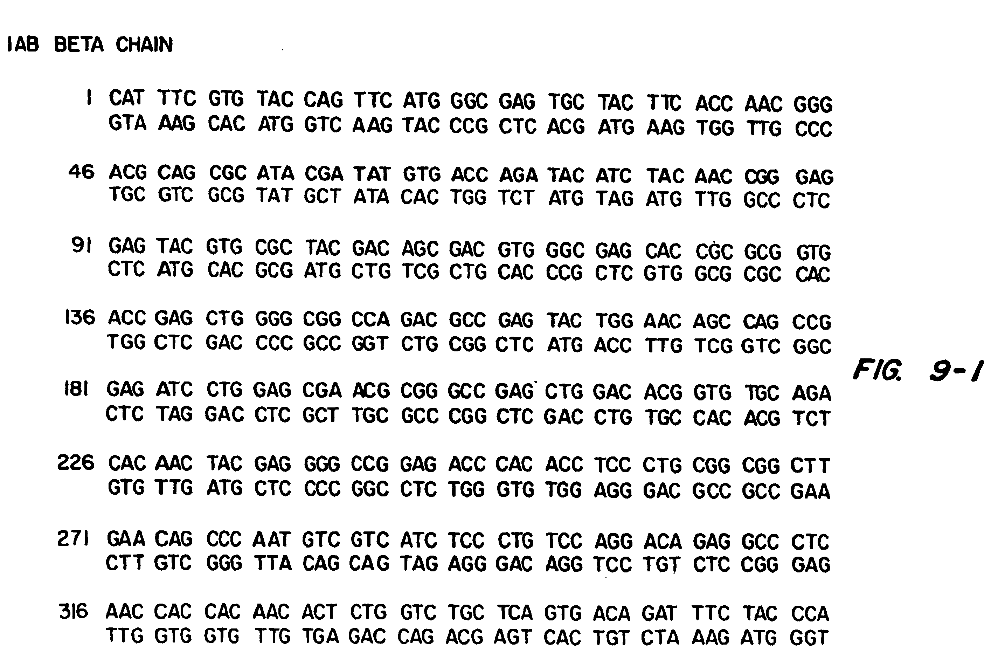

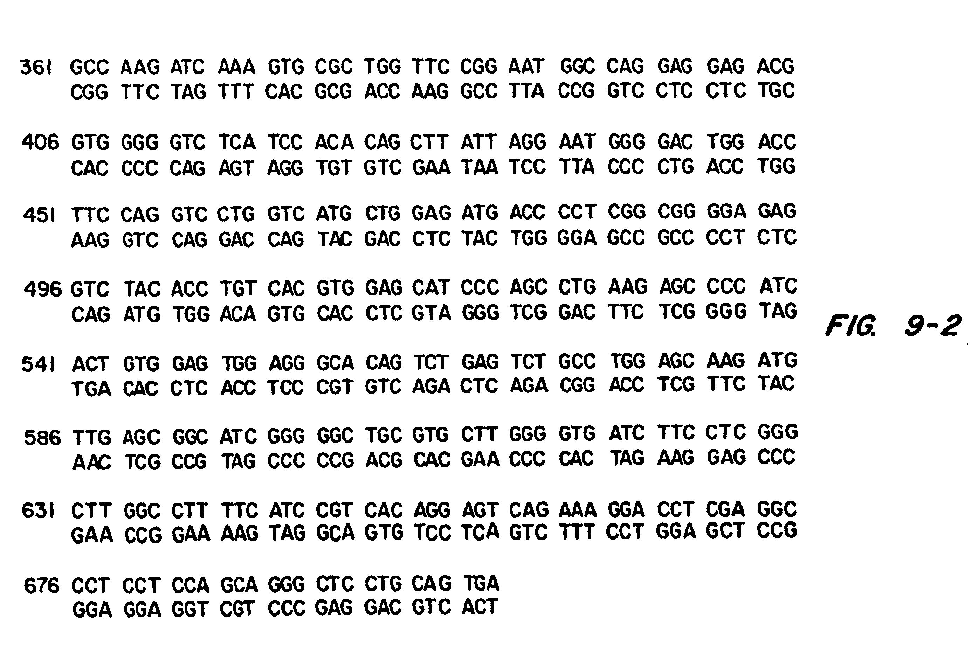

In one protocol, the AChR 195-215 peptide is attached to the N-terminal end of the appropriate MHC molecule. If the recombinant complex is to be used in mice, for example, the AChR peptide may be incorporated into a sequence encoding either the I-A$^b$-alpha or I-A$^b$-beta chain. The sequences encoding these chains are known, and are shown in Figure 8 (alpha chain), and Figure 9 (beta chain); also shown in the figures are restriction enzyme sites and significant domains of the chains. If the AChR peptide is to be incorporated into the beta chain, for example, the oligonucleotide may be inserted as a replacement for the leader peptide. Methods of replacing sequences within polynucleotides are known in the art, examples of which are described in the section on the construction of plasmids.

A similar protocol may be used for incorporation of the AChR peptide into a sequence encoding a peptide derived from the appropriate human HLA antigen. For example, in humans, the haplotype DR2W2 is associated with MG. Hence, the AChR peptide may be incorporated into, for example, a sequence encoding a beta-chain of a DR2 allele. The structural basis in the DR subregion for the major serological specificities DR1-9 are known, as are the sequences encoding the HLA-DR-beta thains from a number of DR haplotypes. See, for e.g., Bell et al. (1987), Proc. Natl. Acad. Sci. USA 84:6234-6238.

As demonstrated above, the autoimmune antigen peptide and the MHC component may be linked via peptide linkages. However, other modes of linkage are obvious to those of skill in the art, and could include, for example, attachment via carbohydrate groups on the glycoproteins, including, e.g., the carbohydrate moieties of the alpha- and/or beta-chains.

Assessment of the Complex

The complexes of the invention can be assayed using an in vitro system or using an in vivo model. In the in vitro system, the complex is incubated with peripheral blood T cells from subjects immunized with, or showing immunity to, the protein or antigen responsible for the condition associated with the peptide of the complex. The successful complexes will induce anergy in syngeneic T cells and prevent proliferation of the T cells even upon stimulation with additional antigen.

In the in vivo system, T cells that proliferate in response to the isolated epitope or to the full length antigen in the presence of APC are cloned. The clones are injected into histocompatible animals which have not been immunized in order to induce the autoimmune disease. Symptoms related to the relevant complex should ameliorate or eliminate the symptoms of the disease.

Either of the types of complexes, i.e., with or without the effector component, may be used. In one mode the treatment is two-fold. The individual is treated with the complex of MHC-encoded antigen-presenting glycoprotein containing an effective portion of the antigen to down-regulate the immune system. Further down-regulation is achieved by treatment with the three component complex with includes the MHC-encoded antigen-presenting glycoprotein, an effective portion of antigen which is specific for the autoimmune disease being treated, and an effector component. In addition, panels of complexes may be used for treatment. For example, if it is suspected that more than one peptide of an antigen is involved in the autoimmune response, and/or if it is suspected that more than one antigen is involved, the individual may be treated with several complexes selected from a panel containing the effective portion of the appropriate MHC-encoded antigen-presenting polypeptides, and effective portions of antigens; these may be with or without effector components.

Administration of a labeled complex permits identification of those portions of the immune system involved in the disease, in diagnostic applications.

Selection of the MHC Complexes for Therapy and/or Diagnosis

In order to select the MHC complexes of the invention which are to be used in the diagnosis or treatment of an individual for an autoimmune disease, the type of MHC antigens which are involved in the presentation of the autoantigen are identified.

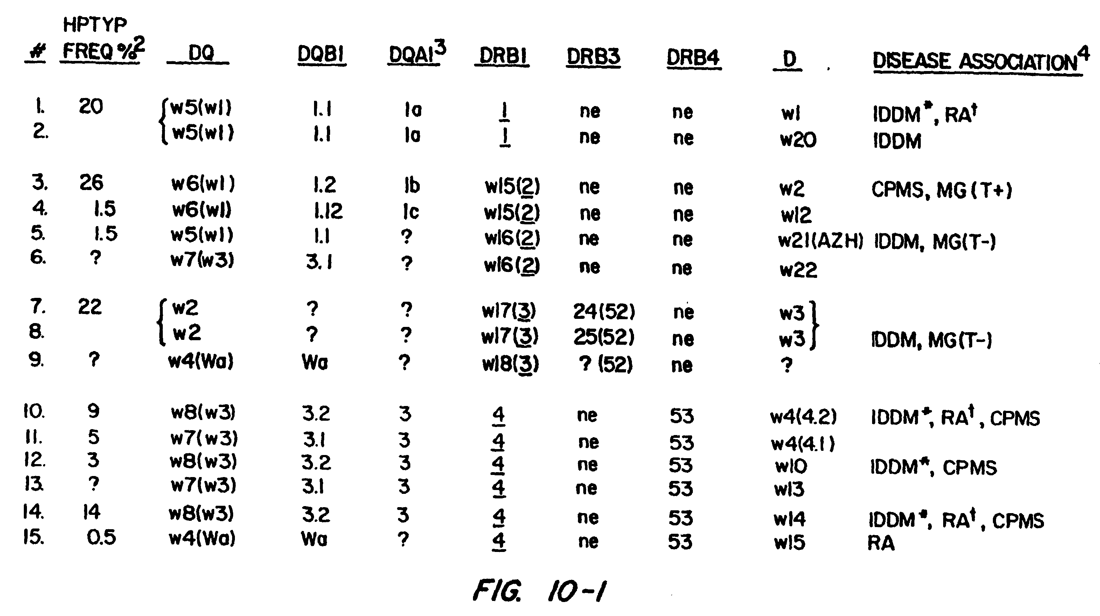

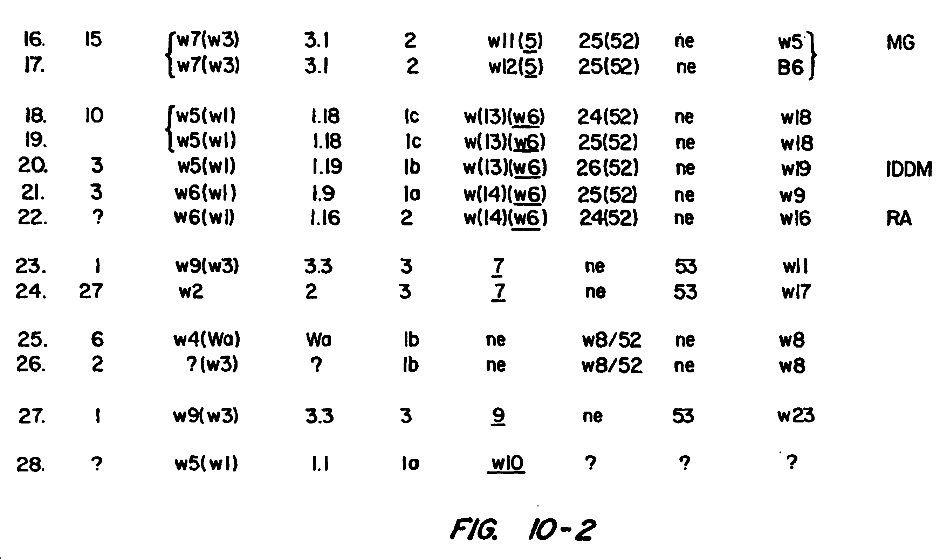

Specific autoimmune dysfunctions are correlated with specific MHC types. A list of the DQ/DR haplotypes in humans and their associations with autoimmune diseases are shown in Figure 10. Methods for identifying which alleles, and subsequently which MHC encoded polypeptides, are associated with an autoimmune disease are known in the art. A method described in EP 286447 is suitable. In this method several steps are followed. First, the association between an MHC antigen and the autoimmune disease is determined based upon genetic studies. The methods for carrying out these studies are known to those skilled in the art, and information on all known HLA disease associations in humans is maintained in the HLA and Disease Registry in Copenhagen. The locus encoding the polypeptide associated with the disease is the one that would bear the strongest association with the disease (See Figure 10).

Second, specific alleles encoding the disease associated with MHC antigen/polypeptide are identified. In the identification of the alleles, it is assumed that the susceptibility allele is dominant. Identification of the allele is accomplished by determining the strong positive association of a specific subtype with the disease. This may be accomplished in a number of ways, all of which are known to those skilled in the art. E.g., subtyping may be accomplished by mixed lymphocyte response (MLR) typing and by primed lymphocyte testing (PLT). Both methods are described in Weir and Blackwell, eds., Handbook of Experimental Immunology. It may also be accomplished by analyzing DNA restriction fragment length polymorphism (RFLP) using DNA probes that are specific for the MHC locus being examined. E.g., Nepom (1986), Annals N.Y. Acad. Sci. 475, 1. Methods for preparing probes for the MHC loci are known to those skilled in the art. See, e.g., Gregersen et al. (1986), Proc. Natl. Acad. Sci. USA 79:5966; Weissman et al. in Medicine in Transition: the Centennial of the University of Illinois College of Medicine (E.P. Cohen, ed. 1981).

The most complete identification of subtypes conferring disease susceptibility is accomplished by sequencing of genomic DNA of the locus, or cDNA to mRNA encoded within the locus. The DNA which is sequenced includes the section encoding the hypervariable regions of the MHC encoded polypeptide. Techniques for identifying specifically desired DNA with a probe, for amplification of the desired region are known in the art, and include, for example, the polymerase chain reaction (PCR) technique.

Once the allele which confers susceptibility to the specific autoimmune disease is identified, the polypeptide encoded within the allele is also identifiable, i.e., the polypeptide sequence may be deduced from the sequence of DNA within the allele encoding it. The MHC antigen complexes of the invention used for diagnosis and/or therapy are derived from the effective portion of the MHC antigen associated with the autoimmune disease state and from an autoimmune antigen associated with the same disease state.

As an example, over 90% of rheumatoid arthritis patients have a haplotype of DR4(Dw4), DR4(Dw14) or DR1 (See Figure 10). It is also known that a target antigen in human rheumatoid arthritis is type-II collagen. Hence, the complexes of the invention used for treatment or diagnosis of an individual with rheumatoid arthritis would include those containing a polypeptide derived from the DR4(Dw4), DR1 and/or DR4(Dw14) which is capable of antigen presentation for disease induction, or incapable of antigen presention for disease suppression, complexed with an effective portion of type-II collagen.

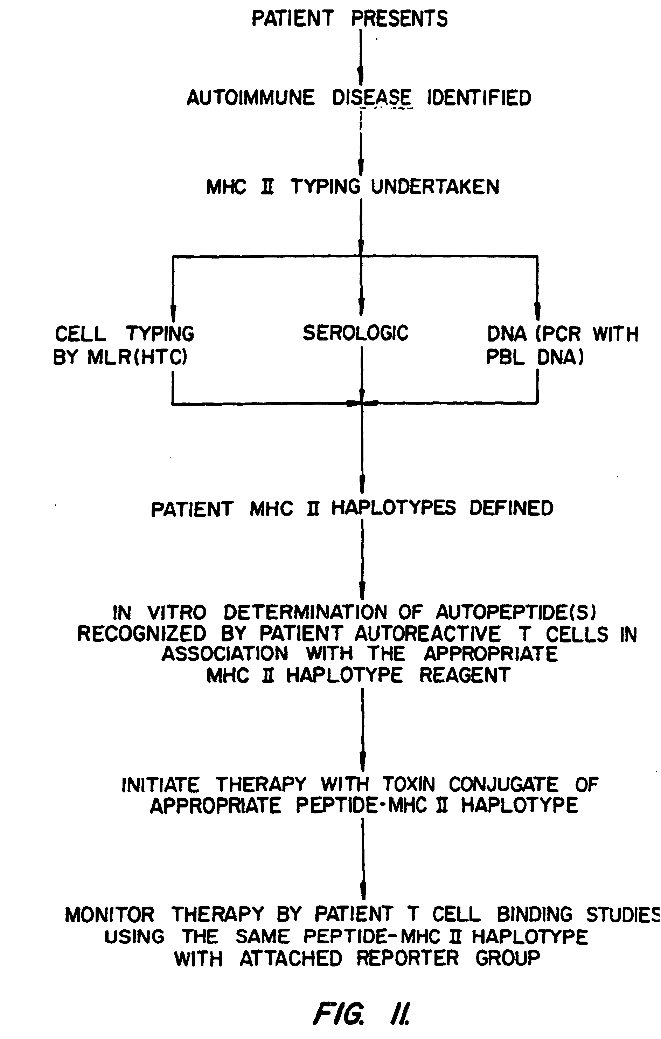

A protocol which may be suitable for the utilization of the complexes of the invention for the diagnosis and/or treatment of an autoimmune disease is depicted in Figure 11. Briefly, an individual having (or susceptible to) an autoimmune disease is identified, and the autoimmune dysfunction is identified. Identification may be by symptomology and/or an examination of family histories. The individual's MHC type is determined by one or more of several methods known in the art, including, for example, cell typing by MLR, by serologic assay, and by DNA analysis (including RFLP and PCR techniques). The individuals T cells are examined in vitro, to determine the autopeptide(s) recognized by autoreactive T cells; this is accomplished utilizing labeled complexes of the invention, described supra, which are of the formula $X^1$ $MHC^2$ peptide, wherein X is a label moiety. After it is determined which complexes target the T cells, the individual is treated with complexes of the invention which are able to suppress the specific autoreactive T cell replication and/or those which kill the autoreactive T cells; these are complexes of the type $MHC^2$ peptide, and, $X^1$ $MHC^2$ peptide (wherein X is a moiety capable of killing the T cell), respectively. Therapy (as determined by the autoreactive T cells remaining) is monitored with T cell binding studies using the labeled complexes of the invention, described supra.

As used herein, the term "individual" encompasses all mammals and all vertebrates which possess basically equivalent MHC systems.

## Model Systems for In vivo Testing

The following is a model system for multiple sclerosis which can be used to evaluate the effects of the complexes of the invention on this condition.

## Experimental Allergic Encephalomyelitis (EAE)

Experimental allergic encephalomyelitis (EAE) is an induced autoimmune disease of the central nervous system which mimics in many respects the human disease of multiple sclerosis (MS). The disease can be induced in many species, including mice and rats.

The disease is characterized by the acute onset of paralysis. Perivascular infiltration by mononuclear cells in the CNS is observed in both mice and rats. Methods of inducing the disease, as well as symptomology, are reviewed in Aranson (1985) in The Autoimmune Diseases (eds. Rose and Mackay, Academic Press, Inc.) pp. 399-427, and in Acha-Orbea et al. (1989), Ann. Rev. Inm. 7:377-405.

One of the genes mediating susceptibility is localized in the MHC class II region (Moore et al. (1980), J. Immunol. 124:1815-1820). The best analyzed encephalitogenic protein is myelin basic protein (MBP), but other encephalitogenic antigens are found in the brain. The immunogenic epitopes have been mapped (see Acha-Orbea et al., supra.). In the PL mouse strains ($H-2^u$) two encephalitogenic peptides in MBP have been characterized: MBP peptide p35-47 (MBP 35-47), and acetylated (MBP 1-9). In humans, preferred autoantigenic peptides for treatment of MS comprise amino aids 84-102 and 148-162 of MBP.

The effect of the invention complexes on ameliorating disease symptoms in individuals in which EAE has been induced can be measured by survival rates, and by the progress of the development of symptoms.

## Formulation and Administration

If the transmembrane region of the MHC subunit is included, the complexes of the invention are conveniently administered after being incorporated in lipid monolayers or bilayers. Typically liposomes are used for this purpose but any form of lipid membrane, such as planar lipid membranes or the cell membrane of a cell (e.g., a red blood cell) may be used. The complexes are also conveniently incorporated into micelles. The data presented in Example 2, below, shows that MHC-peptide complexes comprising dimeric MHC molecules exist primarily as aggregates.

Liposomes can be prepared according to standard methods, as described below. However, if the transmembrane region is deleted, the complex can be administered in a manner conventionally used for peptide-containing pharmaceuticals.

Administration is systemic and is effected by injection, preferably intravenous, thus formulations compatible with the injection route of administration may be used. Suitable formulations are found in Remington's Pharmaceutical Sciences, Mack Publishing Company, Philadelphia, PA, 17th ed. (1985). A variety of pharmaceutical compositions comprising complexes of the present invention and pharmaceutically effective carriers can be prepared. The pharmaceutical compositions are suitable in a variety of drug delivery systems. For a brief review of present methods of drug delivery, see, Langer, Science 249:1527-1533 (1990).

In preparing pharmaceutical compositions of the present invention, it is frequently desirable to modify the complexes of the present invention to alter their pharmacokinetics and biodistribution. For a general discussion of pharmacokinetics, see, Remington's Pharmaceutical Sciences, supra, Chapters 37-39. A number of methods for altering pharmacokinetics and biodistribution are known to one of ordinary skill in the art (see, e.g., Langer, supra). For instance, methods suitable for increasing serum half-life of the complexes include treatment to remove carbohydrates which are involved in the elimination of the complexes from the bloodstream. Preferably, substantially all of the carbohydrate moieties are removed by the treatment. Substantially all of the carbohydrate moieties are removed if at least about 75%, preferably about 90%, and most preferably about 99% of the carbohydrate moieties are removed. Conjugation to soluble macromolecules, such as proteins, polysaccharides, or synthetic polymers, such as polyethylene glycol, is also effective. Other methods include protection of the complexes in vesicles composed of substances such as proteins, lipids (for example, liposomes), carbohydrates, or synthetic polymers.

Liposomes of the present invention typically contain the MHC-peptide complexes positioned on the surface of the liposome in such a manner that the complexes are available for interaction with the T cell receptor. The transmembrane region is usually first incorporated into the membrane at the time of forming the membrane. The liposomes can be used to target desired drugs (e.g. toxins or chemotherapeutic agents) to particular autoreactive T cells. Alternatively, the complexes embedded in the liposome may be used to induce anergy in the targeted cells.

Liposome charge is an important determinant in liposome clearance from the blood, with negatively charged liposomes being taken up more rapidly by the reticuloendothelial system (Juliano, Biochem. Biophys. Res. Commun. 63:651 (1975)) and thus having shorter half-lives in the bloodstream. Liposomes with prolonged circulation half-lives are typically desirable for therapeutic and diagnostic uses. For instance, liposomes which can be maintained from 8, 12, or up to 24 hours in the bloodstream are particularly preferred.

Typically, the liposomes are prepared with about 5-15 mole percent negatively charged phospholipids, such as phosphatidylglycerol, phosphatidylserine or phosphatidylinositol. Added negatively charged phospholipids, such as phosphatidylglycerol, also serve to prevent spontaneous liposome aggregating, and thus minimize the risk of undersized liposomal aggregate formation. Membrane-rigidifying agents, such as sphingomyelin or a saturated neutral phospholipid, at a concentration of at least about 50 mole percent, and 5-15 mole percent of monosialylganglioside, may provide increased circulation of the liposome preparation in the bloodstream, as generally described in U.S. Pat. No. 4, 837,028.

Additionally, the liposome suspension may include lipid-protective agents which protect lipids against free-radical and lipid-peroxidative damages on storage. Lipophilic free-radical quenchers, such as αtocopherol and water-soluble iron-specific chelators, such as ferrioxianine, are preferred.

A variety of methods are available for preparing liposomes, as described in, e.g., Szoka et al., Ann. Rev. Biophys. Bioeng. 9:467 (1980), U.S. Pat. Nos. 4, 235,871, 4,501,728 and 4,837,028. One method produces multilamellar vesicles of heterogeneous sizes. In this method, the vesicle-forming lipids are dissolved in a suitable organic solvent or solvent system and dried under vacuum or an inert gas to form a thin lipid film. If desired, the film may be redissolved in a suitable solvent, such as tertiary butanol, and then lyophilized to form a more homogeneous lipid mixture which is in a more easily hydrated powderlike form. This film is covered with an aqueous solution of the targeted drug and the targeting component and allowed to hydrate, typically over a 15-60 minute period with agitation. The size distribution of the resulting multilamellar vesicles can be shifted toward smaller sizes by hydrating the lipids under more vigorous agitation conditions or by adding solubilizing detergents such as deoxycholate.

The hydration medium contains the targeted drug at a concentration which is desired in the interior volume of the liposomes in the final liposome suspension. Typically the drug solution contains between 10-100 mg/ml of the complexes in a buffered saline solution.

Following liposome preparation, the liposomes may be sized to achieve a desired size range and relatively narrow distribution of liposome sizes. One preferred size range is about 0.2-0.4 microns, which allows the liposome suspension to be sterilized by filtration through a conventional filter, typically a 0.22 micron filter. The filter sterilization method can be carried out on a high through-put basis if the liposomes have been sized down to about 0.2-0.4 microns.

Several techniques are available for sizing liposome to a desired size. One sizing method is described in U. S. Pat. No. 4,737,323. Sonicating a liposome suspension either by bath or probe sonication produces a progressive size reduction down to small unilamellar vesicles less than about 0.05 microns in size. Homogenization is another method which relies on shearing energy to fragment large liposomes into smaller ones. In a typical homogenization procedure, multilamellar vesicles are recirculated through a standard emulsion homogenizer until selected liposome sizes, typically between about 0.1 and 0.5 microns, are observed. In both methods, the particle size distribution can be monitored by conventional laser-beam particle size discrimination.

Extrusion of liposome through a small-pore polycarbonate membrane or an asymmetric ceramic membrane is also an effective method for reducing liposome sizes to a relatively well-defined size distribution. Typically, the suspension is cycled through the membrane one or more times until the desired liposome size distribution is achieved. The liposomes may be extruded through successively smaller-pore membranes, to achieve a gradual reduction in liposome size.

Even under the most efficient encapsulation methods, the initial sized liposome suspension may contain up to 50% or more complex in a free (nonencapsulated) form.

Several methods are available for removing non-entrapped compound from a liposome suspension. In one method, the liposomes in the suspension are pelleted by highspeed centrifugation leaving free compound and very small liposomes in the supernatant. Another method involves concentrating the suspension by ultrafiltration, then resuspending the concentrated liposomes in a replacement medium. Alternatively, gel filtration can be used to separate large liposome particles from solute molecules.

Following the above treatment, the liposome suspension is brought to a desired concentration for use in intravenous administration. This may involve resuspending the liposomes in a suitable volume of injection medium, where the liposomes have been concentrated, for example by centrifugation or ultrafiltration, or concentrating the suspension, where the drug removal step has increased total suspension volume. The suspension is then sterilized by filtration as described above. The liposomes comprising the MHC-peptide complex may be administered parenterally or locally in a dose which varies according to, e.g., the manner of administration, the drug being delivered, the particular disease being treated, etc.

Micelles are commonly used in the art to increase solubility of molecules having nonpolar regions. One of skill will thus recognize that micelles are useful in compositions of the present invention. Micelles comprising the complexes of the invention are prepared according to methods well known in the art (see, e.g., Remington's Pharmaceutical Sciences, supra, Chap. 20). Micelles comprising the complexes of the present invention are typically prepared using standard surfactants or detergents.

Micelles are formed by surfactants (molecules that contain a hydrophobic portion and one or more ionic or otherwise strongly hydrophilic groups) in aqueous solution. As the concentration of a solid surfactant increases, its monolayers adsorbed at the air/water or glass/water interfaces become so tightly packed that further occupancy requires excessive compression of the surfactant molecules already in the two monolayers. Further increments in the amount of dissolved surfactant beyond that concentration cause amounts equivalent to the new molecules to aggregate into micelles. This process begins at a characteristic concentration called "critical micelle concentration".

The shape of micelles formed in dilute surfactant solutions is approximately spherical. The polar head groups of the surfactant molecules are arranged in an outer spherical shell whereas their hydrocarbon chains are oriented toward the center, forming a spherical core for the micelle. The hydrocarbon chains are randomly coiled and entangled and the micellar interior has a nonpolar, liquid-like character. In the micelles of polyoxyethylated nonionic detergents, the polyoxyethlene moieties are oriented outward and permeated by water. This arrangement is energetically favorable since the hydrophilic head groups are in contact with water and the hydrocarbon moieties are removed from the aqueous medium and partly shielded from contact with water by the polar head groups. The hydrocarbon tails of the surfactant molecules, located in the interior of the micelle, interact with one another by weak van der Waals forces.

The size of a micelle or its aggregation number is governed largely by geometric factors. The radius of the hydrocarbon core cannot exceed the length of the extended hydrocarbon chain of the surfactant molecule. Therefore, increasing the chain length or ascending homologous series increases the aggregation number of spherical micelles. For surfactants whose hydrocarbon portion is a single normal alkyl chain, the maximum aggregation numbers consistent with spherical shape are approximately 27, 39, 54, 72, and 92 for $C_8$, $C_{10}$, $C_{12}$, $C_{14}$ and $C_{16}$, respectively. If the surfactant concentration is increased beyond a few percent and if electrolytes are added (in the case of ionic surfactants) or the temperature is raised (in the case of nonionic surfactants), the micelles increase in size. Under these conditions, the micelles are too large to remain spherical and become ellipsoidal, cylindrical or finally lamellar in shape.

Common surfactants well known to one of skill in the art can be used in the micelles of the present invention. Suitable surfactants include sodium laureate, sodium oleate, sodium lauryl sulfate, octaoxyethylene glycol monododecyl ether, octoxynol 9 and PLURONIC F-127° (Wyandotte Chemicals Corp.). Preferred surfactants are nonionic polyoxyethylene and polyoxypropylene detergents compatible with IV injection such as, TWEEN-80°, PLURONIC F-68°, n-octyl-β-D-glucopyranoside, and the like. In addition, phospholipids, such as those described for use in the production of liposomes, may also be used for micelle formation.

Since the MHC subunits of the present invention comprise a lipophilic transmembrane region and a relatively hydrophilic extracellular domain, mixed micelles are formed in the presence of common surfactants or phospholipids and the subunits. The mixed micelles of the present invention may comprise any combination of the subunits, phospholipids and/or surfactants. Thus, the micelles may comprise subunits and detergent, subunits in combination with both phospholipids and detergent, or subunits and phospholipid.

For pharmaceutical compositions which comprise the complexes of the present invention, the dose will vary according to, e.g., the particular complex, the manner of administration, the particular disease being treated and its severity, the overall health and condition of the patient, and the judgment of the prescribing physician. Dosage levels for murine subjects are generally between about 10 μg and about 500 μg. A total dose of between about 50 μg and about 300 μg, is preferred. For instance, in treatments provided over the course of a disease, three 25 μg or 100 μg doses are effective. Total dosages range between about 0.015 and about 15 μg/kg, preferably about 0.15 to about 10 μg/kg.

The pharmaceutical compositions are intended for parenteral, topical, oral or local administration, such as by aerosol or transdermally, for prophylactic and/or therapeutic treatment. The pharmaceutical compositions can be administered in a variety of unit dosage forms depending upon the method of administration. For example, unit dosage forms suitable for oral administration include powder, tablets, pills, and capsules.

Preferably, the pharmaceutical compositions are administered intravenously. Thus, this invention provides compositions for intravenous administration which comprise a solution of the complex dissolved or suspended in an acceptable carrier, preferably an aqueous carrier. A variety of aqueous carriers may be used, e.g., water, buffered water, 0.4% saline, and the like. For instance, phosphate buffered saline (PBS) is particularly suitable for administration of soluble complexes of the present invention. A preferred formulation is PBS containing 0.02% TWEEN-80. These compositions may be sterilized by conventional, well-known sterilization techniques, or may be sterile filtered. The resulting aqueous solutions may be packaged for use as is, or lyophilized, the lyophilized preparation being combined with a sterile aqueous solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The concentration of the complex can vary widely, i.e., from less than about 0.05%, usually at or at least about 1% to as much as 10 to 30% by weight and will be selected primarily by fluid volumes, viscosities, etc., in accordance with the particular mode of administration selected. Preferred concentrations for intravenous administration are about 0.02% to about 0.1% or more in PBS.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating any of the normally employed excipients, such as those carriers previously listed, and generally 10-95% of active ingredient.

For aerosol administration, the complexes are preferably supplied in finely divided form along with a surfactant and propellant. The surfactant must, of course, be nontoxic, and preferably soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride such as, for example, ethylene glycol, glycerol, erythritol, arabitol, mannitol, sorbitol, the hexitol anhydrides derived from sorbitol, and the polyoxyethylene and polyoxypropylene derivatives of these esters. Mixed esters, such as mixed or natural glycerides may be employed. The surfactant may constitute 0.1%-20% by weight of the composition, preferably 0.25-5%. The balance of the composition is ordinarily propellant. Liquefied propellants are typically gases at ambient conditions, and are condensed under pressure. Among suitable liquefied propellants are the lower alkanes containing up to 5 carbons, such as butane and propane; and preferably fluorinated or fluorochlorinated alkanes. Mixtures of the above may also be employed. In producing the aerosol, a container equipped with a suitable valve is filled with the appropriate propellant, containing the finely divided compounds and surfactant. The ingredients are thus maintained at an elevated pressure until released by action of the valve.

The compositions containing the complexes can be administered for therapeutic, prophylactic, or diagnostic applications. In therapeutic applications, compositions are administered to a patient already suffering from a disease, as described above, in an amount sufficient to cure or at least partially arrest the symptoms of the disease and its complications. An amount adequate to accomplish this is defined as "therapeutically effective dose." Amounts effective for this use will depend on the severity of the disease and the weight and general state of the patient. As discussed above, this will typically be between about 0.5 mg/kg and about 25 mg/kg, preferably about 3 to about 15 mg/kg.

In prophylactic applications, compositions containing the complexes of the invention are administered to a patient susceptible to or otherwise at risk of a particular disease. Such an amount is defined to be a "prophylactically effective dose." In this use, the precise amounts again depend on the patient's state of health and weight. The doses will generally be in the ranges set forth above.

In diagnostic applications, compositions containing the appropriately complexes or a cocktail thereof are administered to a patient suspected of having an autoimmune disease state to determine the presence of autoreactive T cells associated with the disease. Alternatively, the efficacy of a particular treatment can be monitored. An amount sufficient to accomplish this is defined to be a "diagnostically effective dose." In this use, the precise amounts will depend upon the patient's state of health and the like, but generally range from 0.01 to 1000 mg per dose, especially about 10 to about 100 mg per patient.

Kits can also be supplied for therapeutic or diagnostic uses. Thus, the subject composition of the present invention may be provided, usually in a lyophilized form in a container. The complexes, which may be conjugated to a label or toxin, or unconjugated, are included in the kits with buffers, such as Tris, phosphate, carbonate, etc., stabilizers, biocides, inert proteins, _e.g._, serum albumin, or the like, and a set of instructions for use. Generally, these materials will be present in less than about 5% wt. based on the amount of complex and usually present in total amount of at least about 0.001% wt. based again on the protein concentration. Frequently, it will be desirable to include an inert extender or excipient to dilute the active ingredients, where the excipient may be present in from about 1 to 99% wt. of the total composition. Where an antibody capable of binding to the complex is employed in an assay, this will usually be present in a separate vial. The antibody is typically conjugated to a label and formulated according to techniques well known in the art.

The following example illustrates, but does not limit, the invention.

Example 1

Down-Regulation of T cells In Vitro With A Complex of Mouse I-A$^k$ and rat MBP Peptide

The efficacy of Class II MHC-peptide complexes in induction of nonresponsiveness or anergy in T cell clones directed against epitopes of myelin basic protein (MBP), known to induce experimental allergic encephalomyelitis in mice, a disease model which mimics human multiple sclerosis, is shown below.

T cell clones AJ1.2 and 4R3.4 prepared by immunization of mice against rat MBP peptide (1-11) and characterized for antigen specificity were obtained from Dr. Pat Jones of Stanford University.

The I-A$^k$ complex with rat NSF peptide was formed utilizing purified mouse I-A$^k$ and synthetic rat MBP peptide (1-13), the sequence for which is known (Zamvil et al. (1986), Nature 324:258260), and which is:

**Ac-ASQKRPSQRHGSK**

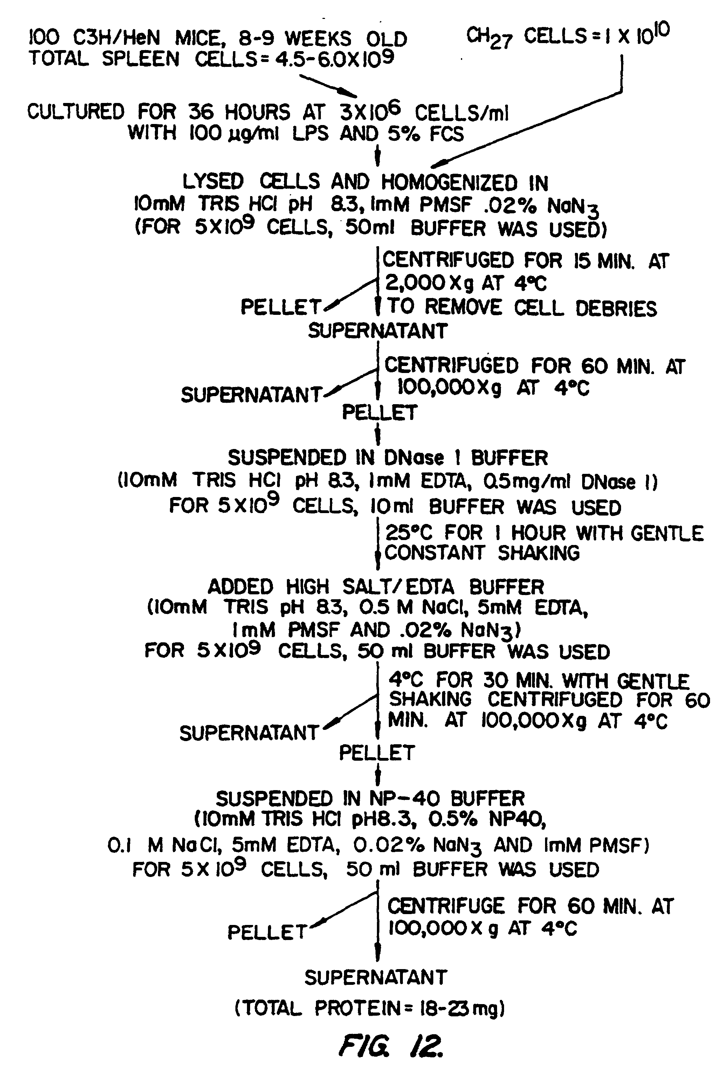

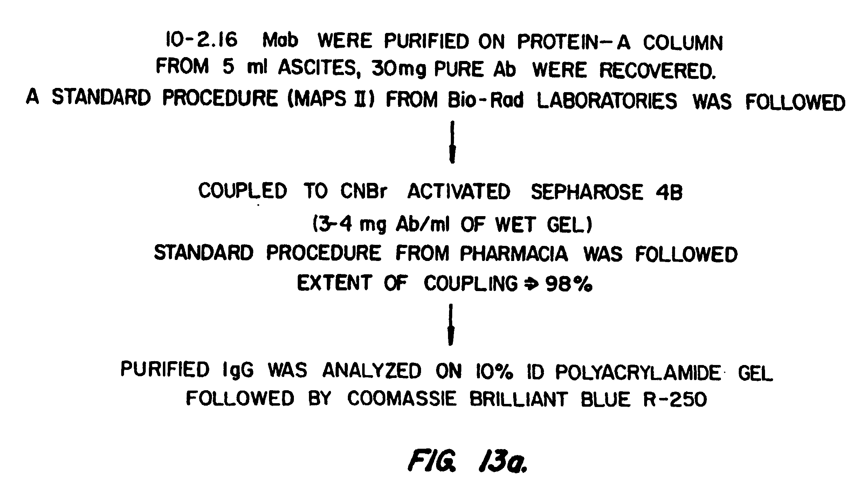

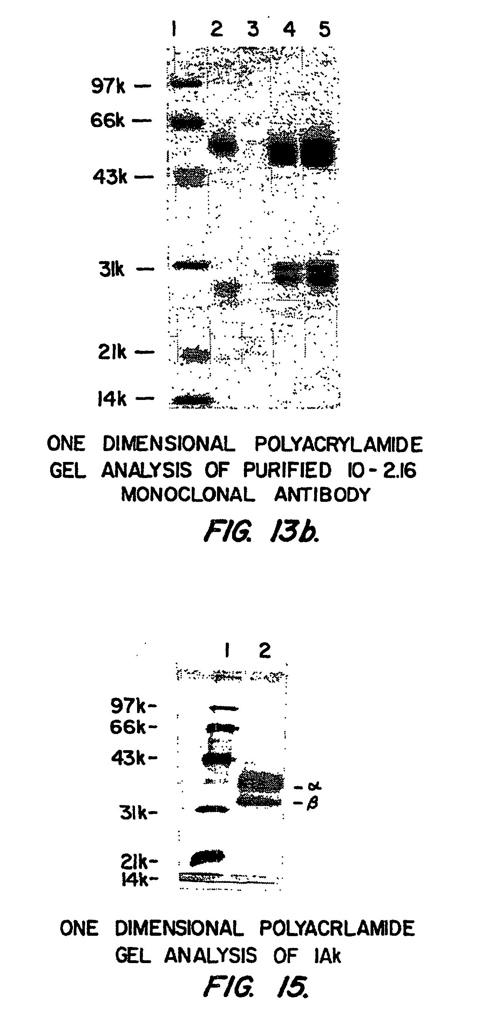

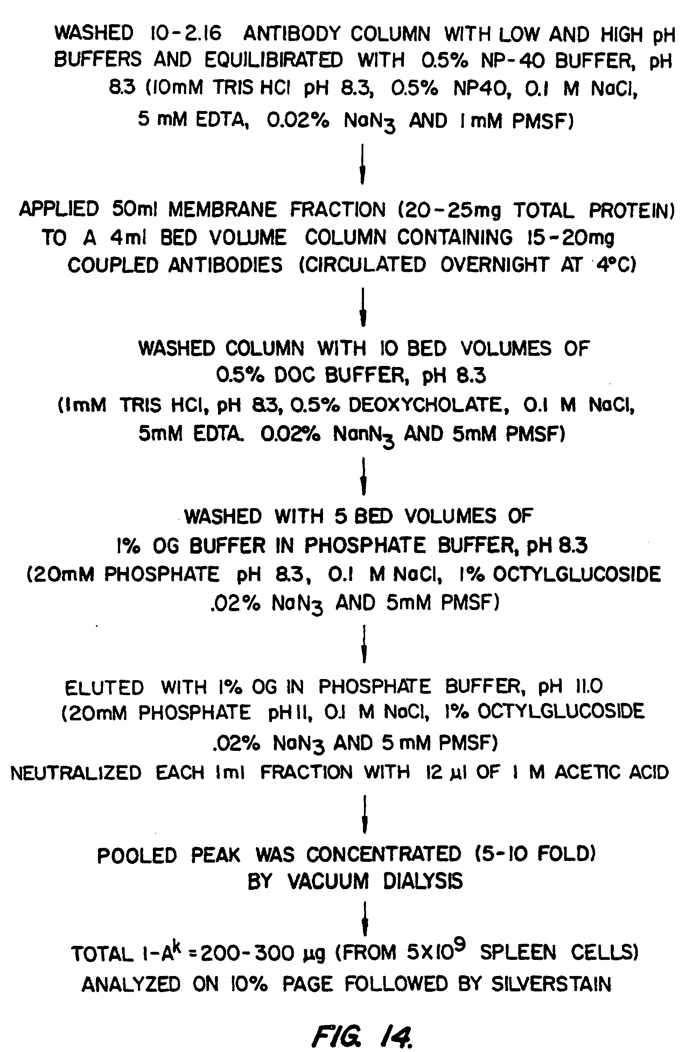

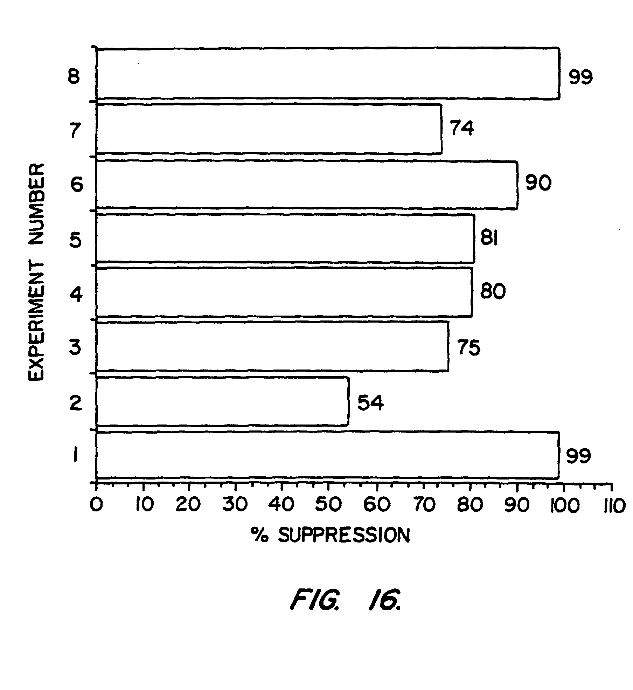

Mouse I-A$^k$ was purified by a modified method based upon Turkewitz et al., supra. Basically, a soluble membrane extract of cells containing I-A$^k$ was prepared using NP-40. I-A$^k$ from the extract was purified by affinity chromatography, using a column containing 10-2.16 antibodies, which had been purified by affinity chromatography on Protein-A, and which were coupled to CNBr activated Sepharose 4b. The preparation schemes for the NP-40 soluble membrane extract, for the purification of 10-2.16 Mab and its coupling to CnBr activated Sepharose 4B, and for the purification of I-A$^k$, are shown in Figures 12, 13a and 14, respectively. Figure 13b is a copy of a polyacrylamide gel showing the Purity of the purified 10-2.16 antibody. The purity of I-A$^k$, as monitored by polyacrylamide gel analysis, is shown in figure 15.

In order to form the complex of I-A$^k$ and rat MBP peptide, ten ug of affinity-purified I-A$^k$ in PBS containing 30 mM octyl glucoside and 50-fold molar excess of HPLC-purified MBP peptide were mixed in a total volume of 125 ul. Samples were incubated at 37°C for 16 hours with constant shaking and were either separated from peptide by G-24 Sephadex desalting for liposome preparation or, for cell studies, were dialyzed against PBS followed by RPMI media for 36 hours at 4°C.

The introduction of the I-A$^K$-MBP peptide complex into liposomes was as follows. A lipid solution consisting of cholesterol:dipalmitoylphosphatidyl choline (DPPC):dipalmitoylphosphatidyl ethanolamine-fluorescein (DPPEF) at a molar ration of 25:75:2 was prepared in chloroform containing 30 mM octyl glucoside (OG). Lipid was dried under vacuum and preformed I-A$^k$-peptide complex in PBS containing 17 mM OG was mixed with dried lipid at a ration of 5:1 (w/w). The mixture was vortexed for 2-3 minutes, cooled to 4°C, and finally dialyzed against PBS followed by RPMI media for 36 at 4°C. In experiments using (125-I)-labeled I-A$^k$, no fluoresceinated lipid was included in the lipid mixture, and the incorporation of I-A$^k$ into liposomes was measured by scintigraphy.

Planar lipid membranes were prepared on sterile 12-mm glass coverslips using 50-100 ul of liposomes containing affinity-purified I-A$^k$ alone or purified I-A$^k$ + MBP(1-13) by the method of Watts et al. (1985), Proc. Natl. Acad. Sci. USA 82:5480-5484. The presence of I-A$^k$ in planar membranes was confirmed by fluorescence microscopy after staining with fluorescent anti-I-A$^k$ anti-body. No fluorescence above background was noted upon staining with fluorescent anti-I-A$^d$.

AJ1.2 and 4R3.4 cells obtained six to eight days after MBP peptide stimulation were washed twice, and the $4 \times 10^5$ cells were added to planar membranes. The plates were incubated for 48-72 hours in 5% $CO_2$ at 37°C and then examined visually for formation of colonies.

The effects of detergent-solubilized Class II molecules were examined by culturing $1 \times 10^5$ AJ1.2 or 4R3.4 cells with 50-100 ul of purified I-A$^k$ alone, purified I-A$^k$ plus MBP(1-13) and medium alone for five hours at 37°C in 5% $CO_2$. Following this incubation the cells were diluted to 900 ul and tested for their ability to respond to antigen-presenting cells (APC) and antigen [MBP(1-13)] in a proliferation assay. Uptake of 3-(4,5-dimethyl-thiazol-2-7')-2,5 diphenyltetrazolium bromide (MTT) was used as an indication of cell proliferation. Although DNA synthesis, usually monitored by $^3$H-thymidine uptake, and the activity of mitochondria, measured by MTT uptake, are different cellular functions, it has been demonstrated that these two activities, monitored three days after initiation of stimulation of spleen cell cultures, tracked each other very well (Molecular Device Application Bulletin Number 011-A, February 9, 1988).

Data are presented as % suppression of proliferation of cells incubated with Class I + Ag compared to cells cultured with medium alone and were calculated by using the formula:

$$\frac{(O.D.)570\ [T\ cells_a + Spleen\ cells + MBP(1\text{-}11)] - (O.D.)\ 570\ [T\ cells_b + Spleen\ cells]}{(O.D.)570\ [T\ cells_c + Spleen\ cells + MBP(1\text{-}13)] - (O.D.)\ 570\ [T\ cells_c + Spleen\ cells]}$$

wherein

T cells$_a$ = T cells preincubated with I-A$^k$ -MBP(1-13) complex

T cells$_b$ = T cells preincubated with I-A$^k$ -MBP(1-13) alone

T cells$_c$ = T cells preincubated with medium.

Since proliferation of cells cultured in the presence of Class II MHC alone was generally equal to cells cultured with medium alone in most studies, this latter number was used in obtaining % suppression. The Standard Deviation of triplicate wells was <10% in the majority of experiments.

Initially two qualitative studies were performed to determine whether pretreatment with I-A$^K$ + MBP(1-13) will alter the binding of T cell clones to planar membranes prepared from liposomes containing I-A$^k$ + MBP(1-13). AJ1.2 cells were used for these studies because they formed characteristic colonies on planar membranes in the presence of MBP(1-13) alone, i.e., without antigen presenting cells (APC). Preincubation of AJ1.2 cells with I-A$^k$ + MBP(1-13) for five hours inhibited the number of colonies formed on planar membranes compared to cells incubated with I-A$^k$ or medium alone. In the second experiment, AJ1.2 cells were incubated with liposomes containing I-A$^k$ + MBP(1-13) or with I-A$^k$ alone for five hours and then added to planar membranes prepared as described above. As noted previously with detergent-solubilized I-A$^k$ MBP(1-13), culturing of cells with liposome containing I-A$^k$ + MBP(1-13) reduced the number of colonies in comparison to cells incubated with liposomes containing I-A$^k$ alone. Although colonies could not be counted accurately, clear differences in their number were evident.

Because these studies did not allow quantitation of the effects of I-A$^k$ + MBP(1-13) on the function of T cell clones, we examined the effects of preincubation with this complex on the proliferation of 4R3.4 or AJ1.2 cells in the presence of APCs and MBP(1-13). Therefore, 4R3.4 or AJ1.2 cells were preincubated with 50-100 ul of I-A$^k$ + MBP (1-13), I-A$^k$, or medium alone for five hours at 37°C. The cells were then diluted to an appropriate concentration and added to APC. Antigen [MBP(1-13)] was added to a final concentration ranging from 13.3 um to 53.2 um.

APC used in the study were prepared from spleens of female A/J mice. Briefly, spleens were removed and single cell suspensions were prepared by gentle teasing between the frosted ends of sterile microscope slides. Red cells were lysed by hypotonic shock. The remaining cells were washed twice with RPMI containing antibiotics and incubated with 10 micrograms/ml mitomycin-C for 1 hour at 37°C. Following this incubation, spleen cells were washed five times with RPMI containing antibiotics, counted, and used as APC's.

Following a 72-hour incubation period of the cells with APC and MBP(1-13), the extent of proliferation was quantitated using MTT uptake. The results of eight such studies are summarized in Figure 16. In studies 1, 3, and 4, 4R3.4 cells were incubated as above. In study 2, 4R3.4 cells were preincubated with liposomes containing I-A$^k$ + MBP (1-13) or I-A$^k$ alone. T cells were then separated from unbound liposomes by centrifugation through a 10% Ficoll solution, washed, and used in proliferation assays. Studies 5 through 8 were carried out with the clone AJ1.2. Cells incubated with I-A$^k$ alone proliferated to the same extent as cells cultured in medium. T cell clones preincubated in this manner did not proliferate in the absence of APC.

The data presented above demonstrate that the complex of Class II MHC + MBP(1-13) induces dramatic nonresponsiveness in T cell clones specific for MBP(1-11). In addition, the data show that this complex was immunologically reactive with, and hence bound to the MBP-stimulated T cell clones.

Preparation and stability of human HLA-DR2-MBP complexes

The optimum pH for maximum binding of human MBP(83-102) peptide to HLA-DR2 was determined to be pH 7. Affinity-purified DR2 from homozygous lymphoblastoid cells was incubated with 10 fold molar excess of radioiodinated MBP(83-102) peptide at 37°C for 48 hours at various pH. The unbound peptide was removed by dialysis and the amount of bound peptide was calculated from the silica gel TLC assay. Samples were also analyzed on reduced and non-reduced polyacrylamide gel. Bands were cut out, counted and the amount of bound peptide was calculated from the specific activity.

The stability of human DR2-MBP(83-102) complex at 4°C was also investigated. Complexes of DR2 and 125I-MBP(83-102) were prepared as described above and stored at 4°C. Every week an aliquot of 1 µl was applied on a silica gel TLC plate in triplicate. Plates were run, developed and the percent dissociation was calculated. Over a period of 42 days, there was no significant dissociation of this complex.

### Example 2

Underline: EBV Transformation of B Cells from an Individual With an Autoimmune Dysfunction

Peripheral blood mononuclear cells (PBMNC) from an individual with an autoimmune dysfunction are isolated by diluting whole blood or buffy 1:1 with sterile phosphate buffered saline (PBS), pH 7.2, layering the suspension on Ficoll-Hypaque, and centrifuging 20 minutes at 1800-2000 RPM in a table top centrifuge. PBMNC present in a band at the interface of the Ficoll-Hypaque and PBS-plasma are harvested with a pipette and washed twice with PBS. Cells are resuspended at $5 \times 10^6$ cells/ml in RPMI 1640 containing 10% fetal serum (FBS), plated in a polystyrene flask and incubated for 1 hour at 37°C to remove monocytes. Non-adherent cells are collected, pelleted by centrifugation and resuspended at $10 \times 10^6$ cells/ml in $Ca^{++}$-$Mg^{++}$ free Dulbecco's PBS containing 15% FBS. AET-SRBC (2% v/v) is mixed 1:1 with PBMC, the mixture is centrifuged for 20 min. at 100 x g, and then incubated on ice for 1 hour. The pellet is gently resuspended, and the suspension centrifuged through Ficoll-Hypaque as described earlier. The band which contains B cells and remaining monocytes is harvested.

Transformation of B cells is with B95-8 cell line (Walls and Crawford in Lymphocytes: A Practical Approach (G.G.B. Klaus ed., IRL Press)). The B95-8 cells are diluted 1:3 in medium, and cultured for 5 days at 37°C. The supernatant is harvested, centrifuged at 250 x g for 15 minutes, and filtered through a 0.45 micron millipore filter. The EBV is then concentrated by centrifugation at 10,000 rpm for 2 hours at 4°C, and the pellet containing the virus is suspended in RPMI 1640 containing 10% FBS, at 1% of the original volume.

In order to transform B cells, the virus stock is diluted 1:9 with culture medium containing $2 \times 10^6$ cells. After the virus is absorbed to the cells for 1-2 hours at 4°C, the cells are centrifuged at 250 x g. The resulting cell pellet is suspended at approximately $0.7 \times 10^6$ to $7.0 \times 10^6$ cells/ml in RPMI 1640 containing 10% FBS. Transformed cells are cloned using standard methods.

The transformed B cells made by the procedure are suitable for the isolation of human MHC glycoproteins.

### Example 3

Induction of EAE in Mice

Adoptive transfer of T cell clones AJ1.2 and 4R3.4, as well as immunization of mice with MBP(1-13) causes mice to develop EAE.

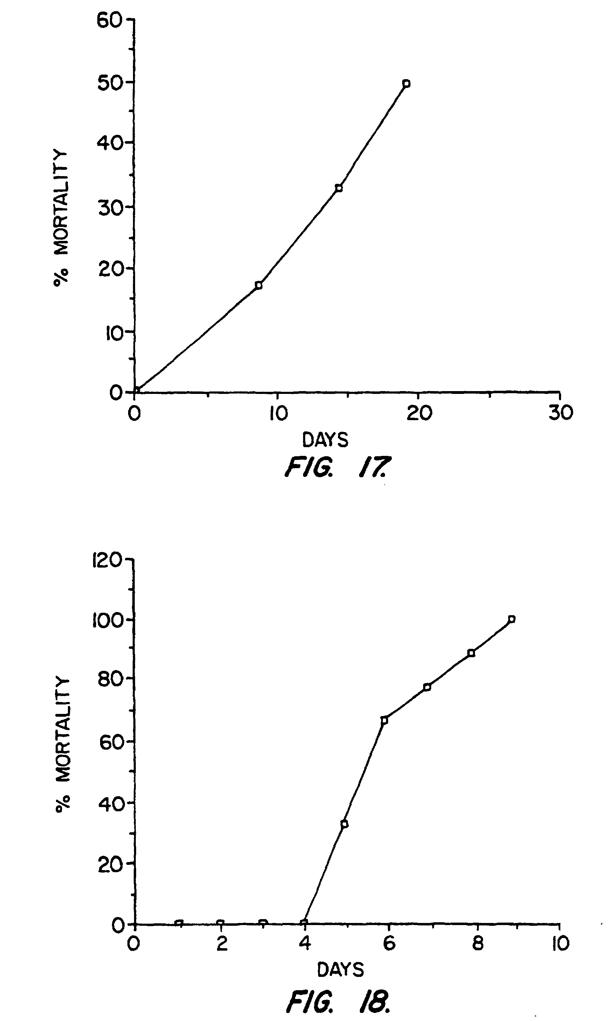

EAE was induced with the peptide using the method for induction of EAE in mice with intact MBP. Briefly, MBP(1-13) was dissolved in PBS and mixed vigorously with complete Freund's adjuvant so as to form a thick emulsion. Female A/J mice were injected with 100 micrograms of this mixture at four sites on the flank. Twenty-four and 72 hours later, 400 ng of pertussis toxin was injected intravenously. Mice are observed daily by two individuals for the development of EAE and mortality. The results in Figure 17 show the development of EAE in mice resulting from immunization with MBP(1-13).

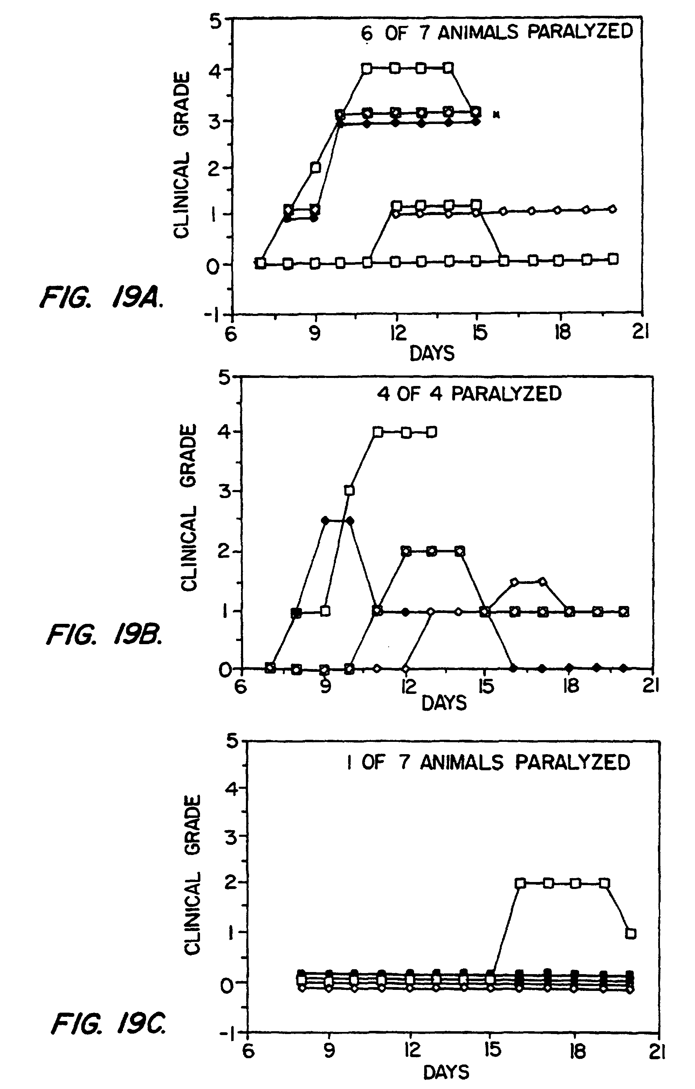

For the adoptive transfer of EAE, T cell clone 4R3.4, obtained from B10A(4R) strain of mice following immunization with MBP(1-11) was used. B10.A(4R) mice were given 350 rad of whole body radiation and then injected with 400 ng pertussis toxin intravenously. Two to three hours later $10 \times 10^6$ 4R3.4 cells, stimulated with MBP(1-13) three days previously, were injected intravenously. These animals were observed twice daily for signs of EAE and mortality. The results of this study are summarized in Fig. 18.

### Example 4

Down-Regulation of EAE by I-A$^s$-MBP(91-103) Complex

This example demonstrates that in vivo therapy with complexes of the present invention results in prevention of passively induced EAE. In addition, the therapy significantly lowered mortality and morbidity in treated animals.

In order to demonstrate that treatment with I-A$^s$/MBP(91-103) complex will prevent the development of EAE following T cell activation, SJL mice were injected with MBP(91-103) reactive T cell blasts in vivo. Briefly, SJL mice 10-12 weeks of age were immunized with 400 µg of MBP(91-103) (Ac-FFKNIVTPRPPP-amide, > 95% purity) in complete Freund's adjuvant on the dorsum. After 10-12 days, regional draining lymph node cells were harvested and cultured in 24 well plates (Falcon) at a concentration $6 \times 10^6$ cells/well in a 1.5 mls of RPMI 1640 media containing 10% fetal bovine serum, 1% penicillin/strepmycin and 50 µg/ml of MBP. Following a 4 day in vitro stimulation, MBP(91-103) reactive T cell blasts were harvested via ficoll-hypaque gradient (Hypaque 1077, Sigma, MO) and washed twice in PBS according to standard techniques. Approximately $1.3$-$1.5 \times 10^7$ cells were injected into each mouse.

Mice that received encephalitogenic MBP(91-103) reactive T cells then received either 100 µg of soluble I-A$^s$/

MBP(91-103) complexes in 100 µl PBS, 100 µg of I-A$^s$/MBP(1-14) (a peptide that is not encephalitogenic in SJL mice) complexes in 100 µl PBS or PBS alone on days 0, 3, and 7 (total dose 300 µg). Animals were observed daily and graded for clinical signs of EAE: grade 1, loss of tail tone; grade 2, hind leg weakness; grade 3, hind leg paralysis; grade 4, moribund; grade 5, death. In accordance with the regulations of the animals care committee, mice that could not feed themselves were sacrificed.

Only one of the seven mice that received I-A$^s$/MBP(91-103) complex developed clinical EAE on day 16 (Figure 19). In contrast, all four animals that received the I-A$^s$/MBP1-14, and six of seven animals that received PBS developed paralysis. In the former group, the mean onset of disease was on days 9.7 and in the latter group it was 9.0 with mean severity of 2.3 and 2.5 respectively.

The inability of other auto-antigens presented by the I-A$^s$ allele to inhibit disease induction was also demonstrated. SJL mice were immunized with the peptide 139-151 of proteolipoprotein (PLP) in complete Freund's adjuvant to induce EAE. SJL mice were immunized with the peptide dissolved in PBS and mixed with complete Freund's adjuvant containing 4 mg/ml Myobacterium tuberculosis H37Ra in a 1:1 ratio. Animals were injected with 152 µg of peptide, a dose found to induce EAE in 100% of the animals, subcutaneously in both abdominal flanks. On the same day, and 48 hours later, all animals were given 400 µg of pertussis toxin intravenously. Mice were treated with PBS, 15 µg of I-A$^s$ alone or 15 µg of I-A$^s$ plus PLP(139-151) on days 1, 4, and 7 after immunization as described above.

As shown in Table 1, animals that received the appropriate I-A$^s$/PLP(139-151) peptide complex were protected from the severe fulminant paralytic disease induced by the immunization with peptide in adjuvant. The was no mortality in the I-A$^s$/PLP peptide treated group. Although all six animals did develop paralysis, the mean severity of animals that were paralyzed was 2.2 and the mean day of onset was 10.6. In contrast, all six animals that received I-A$^s$ complex alone, died with a mean day of onset of 8.2. Five animals died by day 11 and one animal died on day 21. Animals that received saline or no treatment had a mortality of 87% and the average day of onset was 9.2 (p<0.0001 I-A$^s$/PLP (139-151)

TABLE 1

| Treatment Received | No Animals Paralyzed | Mean Severity | Mortality | Day of Onset Paralysis |
|---|---|---|---|---|
| None or Saline | 15 of 15 | 4.7 | 87% | 8 |
| I-A$^s$ complex alone | 6 of 6 | 5 | 100% | 7 |
| I-A$^s$ PLP(139-151) | 6 of 6 | 2.2 | 0 | 10 |

Our observations indicate that in vivo therapy with I-A$^s$/MBP(91-103) complexes (300 µg) results in the prevention of passively induced EAE. In addition, therapy with 45 µg of I-A$^s$/PLP(139-151) significantly lowered the mortality and morbidity in animals that received this therapy.

The complexes of the invention were also tested for the ability to prevent relapse of EAE in mice. In these experiments, SJL mouse received 1.2 x 10$^7$ p91-103 reactive T cells ip on day zero. Initial paralytic signs developed between 8-12 days and all animals recovered by at least 2 clinical grades by day 22. The mice received 50 µgm of MHC complex (with the cognate peptide and without), or PBS iv on days 27, 37, and 47. In experiment 1 mice were observed for 102 days and in Experiment 2, they were observed for 112 days. The results indicated that the complexes with the cognate peptide were significantly more effective than controls in preventing relapses.

Example 5

Increasing Serum Half-life of the Complexes

This example presents data showing that various modifications of the complexes lead to increased serum half-life.

The protocol for these studies was generally as follows: Affinity-purified, soluble MHC molecules were labeled with $^{125}$I by the iodobeads method (Pierce Chemical Co., Rockford, IL). Excess $^{125}$I was removed by dialysis against PBS containing 0.1% neutral detergent. The quality of the labeled protein was assessed by thin layer chromatography, cellulose acetate electrophoresis, and polyacrylamide gel electrophoresis. The MHC glycoprotein was administered by tail vein injection to mice subjected to Lugol's solution in the drinking water at least one day before injection. Blood samples were obtained at different time points. The animals were then sacrificed to obtain organs of interest. Radioactivity in the blood and organ samples was detected in a gamma well counter according to standard techniques.

A. Effect of asialoletuin on serum half-life

IA$^k$ was labeled and administered to mice as described above. The mice were divided into three sets: (1) I-A$^k$ (10 μg i.v.); (2) I-A$^k$ (10 μg i.v.) plus asialoletuin (10 mg i.v.) plus asialoletuin (100 mg i.p.); and (3) I-A$^k$ (10 μg i.v.) plus asialoletuin (10 mg i.p.). Blood was drawn at different time points and the percent of injected dose retained in the blood was calculated.

The mean serum half-life for the three sets was as follows:

Set 1 - 3 min.
Set 2 - 40 min.
Set 3 - 35 min.

B. Effect of liposomes on serum half-life

I-A$^s$ was labeled as described above. The labeled I-A$^s$ molecules were captured inside liposomes by standard procedures (see, e.g., Remingtion's, supra). Ten mice were injected with 10 μq I-A$^s$, as described above, and divided into four sets: (1) I-A$^s$ alone; (2) liposomal I-A$^s$; (3) liposomal I-A$^s$ coinjected with blank liposomes; and (4) blank liposomes plus, 10 minutes later, liposomal I-A$^s$ coinjected with blank liposomes. Blood samples were obtained at different time points after injection, and the percent of injected dose of I-A$^5$ retained in the blood was calculated.

The serum mean half-life of the three sets was as follows:

Set 1 - 2 min.
Set 2 - 7 min.
Set 3 - 10 min.
Set 4 - 60 min.

C. Effect of periodate/cyanoborohydride treatment on serium half-life

I-A$^k$ in a phosphate buffer containing 3mM taurodeoxycholate at pH 7.5 was labeled as described above. The labeled molecules were subjected to periodate oxidation and cyanoborohydride reduction for 5 or 21 hours at 4°C, using 20 mM sodium periodate and 40 mM cyanoborohydride (final concentrations) in 0.1 M acetate buffer at pH 5.5. The reaction was quenched by addition of ethylene glycol (final concentration 0.7%). The treated I-A$^k$ was purified by dialysis and administered (10 μg i.v.) to mice, as described above. Nine mice were divided into three sets: (1) I-A$^k$ (untreated); (2) I-A$^k$ (5 hr. treatment); and (3) I-A$^k$ (21 hr. treatment). Blood samples were obtained at different time points and the percent of injected dose of I-A$^k$ retained in the blood was calculated.

The serum half-life of the three sets was as follows:

Set 1 - 4 min.
Set 2 - 7 min.
Set 3 - 70 min.

Example 6

The results presented below demonstrate that MHC-peptide complexes of the invention exist primarily as aggregates in the absence of detergent.

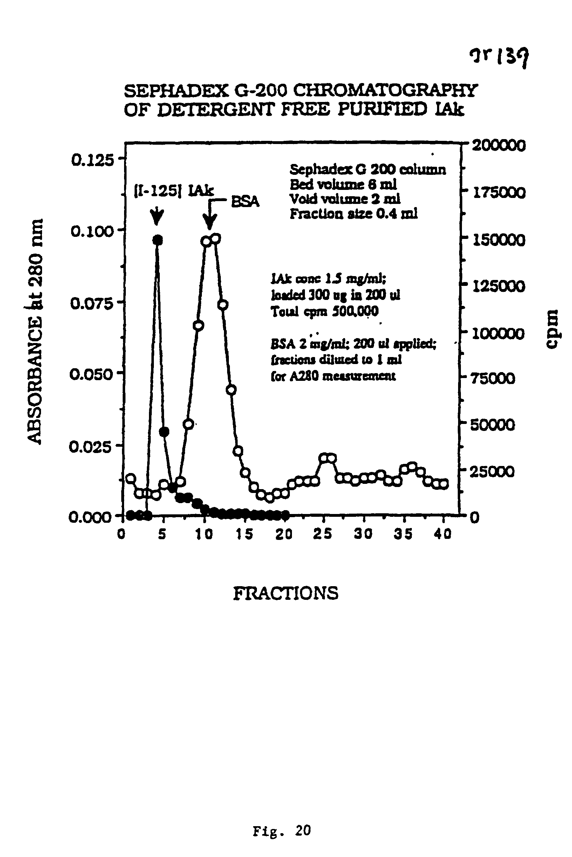

An IA$^k$-P complex labeld with $^{125}$I was prepared as described above. The complex (1.5 mg/ml) was dialysed extensively against phosphate buffered saline (PBS) to remove detergent and loaded on a 6 ml b.v. Sephadex-G200 column (fractionation size 5000-600,000). The results presented in Figure 20 show that the aggregated complexes pass through the column with the void volume and thus have a molecular weight greater than 600,000.

The same dialyzed IA$^k$ complex (1.5 mg/ml) was also centrifuged and the pellet was counted. To do this, 200 μl of complex (300 μg) was diluted in 5 ml PBS and centrifuged in a fixed angle rotor at 100,000 xg for 60 minutes. The results are given in Table 2, below.

TABLE 2 -

| DETECTION OF COMPLEX AGGREGATION BY HIGH SPEED SPIN | | | | |
|---|---|---|---|---|
| EXP # | Starting cpm | cpm in pellet | cpm in sup | % aggreg. |
| 1 | 514,576 | 317,717 | 205,797 | 60.68 |
| 2 | 519,340 | 321,304 | 209,108 | 60.57 |

The results of the chromatography and centrifugation experiments both show that MHC-peptide complexes exist largely in aggregated or micellar form. These results strongly indicate that the single subunit complexes of the present invention are also aggregated or in micellar form, in the absence of detergent.

The results described in the Examples, above, demonstrate the ability of the complexes of the present invention to treat autoimmune disease in vivo. These data in combination with the in vitro data showing induction of anergy establish the effectiveness of the claimed complexes.

**Claims**

1. A substantially pure MHC-peptide complex consisting of an antigenic peptide consisting of residues 84-102 of human MBP or a segment of 8 to 18 amino acids thereof, and an isolated MHC component having an antigen binding site, wherein the antigenic peptide is associated with the antigen binding site.

2. A complex of claim 1, wherein the peptide is noncovalently associated with the antigen binding site.

3. A complex of claim 1, wherein the MHC component is soluble.

4. A complex of claim 1, wherein an epitope on the peptide is recognized by an autoreactive T cell associated with multiple sclerosis.

5. A complex of claim 1, wherein the peptide consists of residues 84-102 of human MBP.

6. A complex of claim 1, wherein the MHC component is Class II MHC.

7. A complex of claim 1, wherein the MHC component is isolated from human lymphoblastoid cells.

8. A pharmaceutical composition comprising a pharmaceutically acceptable carrier and the MHC-peptide complex of claim 1.

9. A pharmaceutical composition of claim 10, wherein the complex is embedded in a liposome.

10. A pharmaceutical composition of claim 10, wherein the complex is substantially free of carbohydrate moieties.

11. A pharmaceutical composition of claim 10, wherein the concentration of the complex is between 0.02% and 1% by weight.

12. A pharmaceutical composition of claim 10, wherein the pharmaceutically acceptable carrier is phosphate buffered saline.

13. A method for preparing a complex consisting of an antigenic peptide consisting of residues 84-102 of human MBP or a segment of 8 to 18 amino acids thereof, and an isolated MHC component having an antigen binding site, the method comprising:

    isolating the MHC component from a cell producing the component;
    contacting the MHC component with the peptide such that the peptide is coupled to the antigen binding site; and
    removing excess peptide not coupled to the antigen binding site.

**14.** A method of claim 13 wherein the step of removing excess peptide is carried out by ultrafiltration.

**15.** A method of claim 13 further comprising the step of dialyzing the complex in the presence of lipids to form liposomes.

**16.** A method of claim 13 wherein the peptide is noncovalently bound to the antigen binding site.

**17.** A method of claim 13 wherein the MHC component is a class II glycoprotein of the major histocompatibility complex.

**Patentansprüche**

**1.** Im Wesentlichen reiner MHC-Peptid-Komplex, bestehend aus einem Antigen-Peptid, das aus den Resten 84-102 von Human-MBP oder einem Segment von 8 bis 18 Aminosäuren davon besteht, und aus einer isolierten MHC-Komponente, die eine Antigen-Bindungsstelle aufweist, worin das Antigen-Peptid mit der Antigen-Bindungsstelle assoziiert ist.

**2.** Komplex nach Anspruch 1, worin das Peptid nichtkovalent mit der Antigen-Bindungsstelle assoziiert ist.

**3.** Komplex nach Anspruch 1, worin die MHC-Komponente löslich ist.

**4.** Komplex nach Anspruch 1, worin ein Epitop auf dem Peptid von einer autoreaktiven T-Zelle erkannt wird, die mit multiper Sklerose in Zusammenhang steht.

**5.** Komplex nach Anspruch 1, worin das Peptid aus den Resten 84-102 von Human-MBP besteht.

**6.** Komplex nach Anspruch 1, worin die MHC-Komponente ein Klasse II-MHC ist.

**7.** Komplex nach Anspruch 1, worin die MHC-Komponente aus Human-Lymphoblast-Zellen isoliert ist.

**8.** Pharmazeutische Zusammensetzung, umfassend einen pharmazeutisch annehmbaren Träger und den MHC-Peptidkomplex nach Anspruch 1.

**9.** Pharmazeutische Zusammensetzung nach Anspruch 10, worin der Komplex in ein Liposom eingebettet ist.

**10.** Pharmazeutische Zusammensetzung nach Anspruch 10, worin der Komplex im Wesentlichen frei von Kohlenhydratgruppen ist.

**11.** Pharmazeutische Zusammensetzung nach Anspruch 10, worin die Konzentration des Komplexes zwischen 0,02 und 1 Gew.-% liegt.

**12.** Pharmazeutische Zusammensetzung nach Anspruch 10, worin der pharmazeutisch annehmbare Träger Phosphat-gepufferte Kochsalzlösung ist.

**13.** Verfahren zur Herstellung eines Komplexes, bestehend aus einem Antigen-Peptid, das aus den Resten 84-102 von Human-MBP oder einem Segment von 8 bis 18 Aminosäuren davon besteht, und aus einer isolierten MHC-Komponente, die eine Antigen-Bindungsstelle aufweist, umfassend:

das Isolieren der MHC-Komponente aus einer die Komponente produzierenden Zelle;
das In-Kontakt-Bringen der MHC-Komponente mit dem Peptid, so dass das Peptid an die Antigen-Bindungsstelle gebunden wird; und
das Entfernen von überschüssigem, nicht an die Antigen-Bindungsstelle gebundenem Peptid.

**14.** Verfahren nach Anspruch 13, worin der Schritt des Entfernens von überschüssigem Peptid durch Ultrafiltration durchgeführt wird.

**15.** Verfahren nach Anspruch 13, das weiters den Schritt des Dialysierens des Komplexes in Gegenwart von Lipiden umfasst, um Liposome zu bilden.

**16.** Verfahren nach Anspruch 13, worin das Peptid nichtkovalent an die Antigen-Bindungsstelle gebunden wird.

**17.** Verfahren nach Anspruch 13, worin die MHC-Komponente ein Klasse II-Glykoprotein des Haupt-Histokompatibilitätskomplexes ist.

**Revendications**

**1.** Complexe CMH-peptide sensiblement pur consistant en un peptide antigénique consistant en résidus 84-102 de MBP humaine ou un segment de 8 à 18 acides aminés de celle-ci et un composant de CMH isolé ayant un site de liaison de l'antigène, où le peptide antigénique est associé au site de liaison de l'antigène.

**2.** Complexe de la revendication 1, où le peptide est associé de manière non convalente au site de liaison de l'antigène.

**3.** Complexe de la revendication 1, où le composant MHC est soluble.

**4.** Complexe de la revendication 1, où un épitope sur le peptide est reconnu par une cellule T autoréactive associée à la sclérose multiple.

**5.** Complexe de la revendication 1, où le peptide consiste en résidus (80-102) de MBP humaine.

**6.** Complexe de la revendication 1, où le composant CMH est CMH Classe II.

**7.** Complexe de la revendication 1, où le composant CMH est isolé de cellules de lymphoblastoïde humain.

**8.** Composition pharmaceutique comprenant un support pharmaceutiquement acceptable et le complexe CMH-peptide de la revendication 1.

**9.** Composition pharmaceutique de la revendication 10, où le complexe est noyé dans un liposome.

**10.** Composition pharmaceutique de la revendication 10, où le complexe est sensiblement exempt de fractions carbohydrate.

**11.** Composition pharmaceutique de la revendication 10, où la concentration du complexe est entre 0,02% et 1% poids.

**12.** Composition pharmaceutique de la revendication 10, où le support pharmaceutiquement acceptable est une solution saline tamponnée phosphate.

**13.** Méthode de préparation d'un complexe consistant en un peptide antigénique consistant en résidus 84-102 de MBP humaine ou un segment de 8 à 18 acides aminés de celle-ci et un composant CMH isolé ayant un site de liaison de l'antigène, la méthode comprenant :

isolement du composant CMH d'une cellule produisant le composant ;
la mise en contact du composant CMH avec le peptide de façon que le peptide soit couplé au site de liaison de l'antigène ; et
l'enlèvement du peptide en excès non couplé au site de liaison de l'antigène.

**14.** Méthode de la revendication 13 où l'étape d'enlèvement du peptide en excès est effectuée par ultrafiltration.

**15.** Méthode de la revendication 13 comprenant de plus l'étape de dialyser le complexe en présence de lipides pour former des liposomes.

**16.** Méthode de la revendication 13 où le peptide est lié de manière non covalente au site de liaison de l'antigène.

**17.** Méthode de la revendication 13 où le composant CMH est une glycoprotéine classe II du complexe majeur d'histocompatibilité.

LABEL OR TOXIN

PEPTIDE

T CELL RECEPTOR

AUTOREACTIVE T CELL

MHC CLASS I OR CLASS II

FITC

PEPTIDE

T CELL RECEPTOR

AUTOREACTIVE T CELL

**FIG. I.**

N

N

$\alpha_1$

$\beta_1$

S-S

$\alpha_2$

$\beta_2$

S-S

S-S

—S–S—

Lys    Lys

**FIG. 3.**

N

N

N

$\alpha_1$

$\alpha_1$

$\beta_1$

S-S

$\beta_1$

S-S

$\alpha_2$

S-S

S-S

$\beta_2$

Lys

Lys

**FIG. 4.**

*FIG. 2a.*

FIG. 2b.

FIG. 5.

```
                                                              -I      I
Leu Leu Leu Phe Ser Cys Cys Gly Leu Val Leu Gly Ser Glu His Glu Thr Arg
CUA CUG UUA UUU UCG UGU UGU GGU CUG GUA CUA GGU UCU GAA CAU GAA ACA CGU
                    -20                              -i  i
         10                                              20
Leu Val Ala Asn Leu Leu Glu Asn Tyr Asn Lys Val Ile Arg Pro Val Glu His
UUG GUU GCU AAU UUA UUA GAA AAU UAU AAC AAG GUG AUU CGU CCA GUG GAG CAU
 20                          40                          60
                    30
His Thr His Phe Val Asp Ile Thr Val Gly Leu Gln Leu Ile Gln Leu Ile Ser
CAC ACC CAC UUU GUA GAU AUU ACA GUG GGG CUA CAG CUG AUA CAA CUC AUC AGU
 80                              100                         120
                         50
Val Asp Glu Val Asn Gln Ile Val Glu Thr Asn Val Arg Leu Arg Gln Gln Trp
GUG GAU GAA GUA AAU CAA AUU GUG GAA ACA AAU GUG CGC CUA AGG CAG CAA UGG
                    140                          160                    180
                              70
Ile Asp Val Arg Leu Arg Trp Asn Pro Ala Asp Tyr Gly Gly Ile Lys Lys Ile
AUU GAU GUG AGG CUU CGC UGG AAU CCA GCC GAU UAU GGU GGA AUU AAA AAG AUC
                    200                          220
   80                                     90
Arg Leu Pro Ser Asp Asp Val Trp Leu Pro Asp Leu Val Leu Tyr Asn Asn Ala
AGA CUG CCU UCU GAU GAU GUU UGG CUG CCA GAU UUA GUU CUG UAC AAC AAU GCU
      240                          260                         280
```

## FIG. 6-1

EP 0 630 255 B1

EP 0 630 255 B1

```
              100                                              110
Asp Gly Asp Phe Ala Ile Val His Met Thr Lys Leu Leu Leu Asp Tyr Thr Gly
GAU GGU GAU UUU GCC AUU GUU CAC AUG ACC AAA CUG CUU UUG GAU UAU ACG GGA
              300                                320                  340

              120                                         130
Lys Ile Met Trp Thr Pro Pro Ala Ile Phe Lys Ser Tyr Gyx Glu Ile Ile Val
AAA AUA AUG UGG ACA CCU CCA GCA AUC UUC AAA AGC UAU UGU GAA AUU AUU GUA
                   360                           380

                        140                                       150
Thr His Phe Pro Phe Asp Gln Gln Asn Cys Thr Met Lys Leu Gly Ile Trp Thr
ACA CAU UUC CCA UUU GAU CAA CAA AAU UGC ACU AUG AAG UUG GGA AUC UGG ACG
    400                           420                       440

                             160
Tyr Asp Gly Thr Lys Val Ser Ile Ser Pro Glu Ser Asp Arg Pro Asp Leu Ser
UAC GAU GGG ACA AAA GUU UCC AUA UCC CCG GAA AGU GAC CGU CCG GAU CUG AGU
         460                           480                       500

    170                                    180
Thr Phe Met Glu Ser Gly Glu Trp Val Met Lys Asp Tyr Arg Gly Trp Lys His
ACA UUU AUG GAA AGU GGA GAG UGG GUA AUG AAA GAU UAU CGU GGA UGG AAG CAC
              520                           540

         190                                     200
Trp Val Tyr Tyr Thr Cys Cys Pro Asp Thr Pro Tyr Leu Asp Ile Thr Tyr His
UGG GUG UAU UAU ACC UGC UGU CCU GAC ACU CCU UAC CUG GAU AUC ACC UAC CAU
560                           580                       600
```

## FIG. 6-2

EP 0 630 255 B1

```
                  210                                               220
Phe  Ile  Met  Gln  Arg  Ile  Pro  Leu  Tyr  Phe  Val  Val  Asn  Val  Ile  Ile  Pro  Cys
UUU  AUC  AUG  CAG  CGU  AUU  CCU  CUU  UAU  UUU  GUU  GUG  AAU  GUC  AUC  AUU  CCU  UGU
         620                                     640                      660


Leu  Leu  Phe  Ser  Phe  Leu  Thr  Gly  Leu  Val  Phe  Tyr  Leu  Pro  Thr  Asp  Ser  Gly
CUG  CUU  UUU  UCA  UUU  UUA  ACU  GGA  UUA  GUA  UUU  UAC  UUA  CCA  ACU  GAU  UCA  GGU
              680                                     700                           720
                                 250
Glu  Lys  Met  Thr  Leu  Ser  Ile  Ser  Val  Leu  Leu  Ser  Leu  Thr  Val  Phe  Leu  Leu
GAG  AAG  AUG  ACU  UUG  AGU  AUU  UCC  GUU  UUG  CUG  UCU  CUG  ACU  GUG  UUC  CUU  CUG
                             740                              760

     260                                          270
Val  Ile  Val  Glu  Leu  Ile  Pro  Ser  Thr  Ser  Ser  Ala  Val  Pro  Leu  Ile  Gly  Lys
GUU  AUU  GUU  GAG  CUG  AUC  CCC  UCA  ACU  UCC  AGC  GCU  GUG  CCU  UUG  AUU  GGC  AAA
        780                                  800                            820
             280                                          290
Tyr  Met  Leu  Phe  Thr  Met  Ile  Phe  Val  Ile  Ser  Ser  Ile  Ile  Ile  Thr  Val  Val
UAC  AUG  CUU  UUU  ACA  AUG  AUU  UUU  GUC  AUC  AGU  UCA  AUC  AUC  AUU  ACU  GUU  GUU
              840                                     860                           880
                  300                                          310
Val  Ile  Asn  Thr  His  His  Arg  Ser  Pro  Ser  Thr  His  Thr  Met  Pro  Gln  Trp  Val
GUA  AUU  AAU  ACU  CAC  CAU  CGC  UCU  CCA  AGU  ACA  CAU  ACA  AUG  CCA  CAA  UGG  GUA
                      900                                  920
```

*FIG. 6-3*

EP 0 630 255 B1

```
                        320                                                      330
Arg Lys Ile Phe Ile Asp Thr Ile Pro Asn Val Met Phe Phe Ser Thr Met Lys
CGA AAG AUC UUU AUU GAU ACU AUA CCC AAU GUU AUG UUU UUC UCA ACA AUG AAA
     940                          960                          980

                                  340
Arg Ala Ser Lys Glu Lys Gln Glu Asn Lys Ile Phe Ala Asp Asp Ile Asp Ile
CGA GCU UCU AAG GAA AAG CAA GAA AAU AAG AUA UUU GCU GAU GAC AUU GAU AUC
         1,000                        1,020                        1,040

    350                                   360
Ser Asp Ile Ser Gly Lys Gln Val Thr Gly Glu Val Ile Phe Gln Thr Pro Leu
UCU GAC AUU UCU GGA AAG CAA GUG ACA GGA GAA GUA AUU UUU CAA ACA CCU CUC
             1,060                        1,080

        370                                       380
Ile Lys Asn Pro Asp Val Lys Ser Ala Ile Glu Gly Val Lys Tyr Ile Ala Glu
AUU AAA AAU CCA GAU GUC AAA AGU GCU AUU GAG GGA GUC AAA UAU AUU GCA GAG
1,100                         1,120                        1,140

                    390                                           400
His Met Lys Ser Asp Glu Glu Ser Ser Asn Ala Ala Glu Glu Trp Lys Tyr Val
CAC AUG AAG UCU GAU GAG GAA UCA AGC AAU GCU GCA GAG GAA UGG AAA UAU GUU
         1,160                        1,180                        1,200

                            410                                       420
Ala Met Val Ile Asp His Ile Leu Leu Cys Val Phe Met Leu Ile Cys Ile Ile
GCA AUG GUG AUU GAU CAC AUU CUG CUG UGU GUC UUC AUG CUG AUU UGU AUA AUU
             1,220                        1,240                        1,260

                    430
Gly Thr Val Ser Val Phe Ala Gly Arg Leu Ile Glu Leu Ser Gln Gly Gly
GGU ACA GUU AGC GUG UUU GCU GGC CGU CUC AUU GAA CUC AGU CAA GAG GGC UAA
             1,280                                1,300
```

## FIG. 6-4

EP 0 630 255 B1

FIG. 7

                    (-)                                    His -Gly
N-Ac-Ala-Ser-Ala-Gln-Lys-Arg-Pro-Ser-Gln-Arg-Ser-Lys-Tyr-Leu-Ala-
                                              10

Thr
Ser-Ala-Ser-Thr-Met-Asp-His-Ala-Arg-His-Gly-Phe-Leu-Pro-Arg-His-
                    20                                              30

                                        Ile                    Gly
Arg-Asp-Thr-Gly-Ile-Leu-Asp-Ser-Leu-Gly-Arg-Phe-Phe-Gly-Ser-Asp-
                              40

                                                    Ser
Arg-Gly-Ala-Pro-Lys-Arg-Gly-Ser-Gly-Lys-Asp-Gly-His-His-Ala-Ala-Arg-
          50                                    60

     Ala                                    Ser  (-)                Thr
Thr-Thr-His-Tyr-Gly-Ser-Leu-Pro-Gln-Lys-Ala-Gln-Gly-His-Arg-Pro-Gln-
                    70                                              80

Asp-Glu-Asn-Pro-Val-Val-His-Phe-Phe-Lys-Asn-Ile-Val-Thr-Pro-Arg-Thr-
                              90

                                        Me
Pro-Pro-Pro-Ser-Gln-Gly-Lys-Gly-Arg-Gly-Leu-Ser-Leu-Ser-Arg-Phe-Ser-
          100                                    110

                         Arg
Trp-Gly-Ala-Glu-Gly-Gln-Lys-Pro-Gly-Phe-Gly-Tyr-Gly-Gly-Arg-Ala-Ser-
                    120                                              130

                                        Phe         Val
Asp-Tyr-Lys-Ser-Ala-His-Lys-Gly-Leu-Lys-Gly-His-Asp-Ala-Gln-Gly-Thr-
                              140

Leu-Ser-Lys-Ile-Phe-Lys-Leu-Gly-Gly-Arg-Asp-Ser-Arg-Ser-Gly-Ser-Pro-
150                                    160

Met-Ala-Arg-Arg-COOH
          170

IAB ALPHA CHAIN

```
  I  AAT  TCA  TGC  CGC  GCA  GCA  GAG  CTC  TGA  TTC  TGG  GGG  TCC  TCG  CCC
     TTA  AGT  ACG  GCG  CGT  CGT  CTC  GAG  ACT  AAG  ACC  CCC  AGG  AGC  GGG


 46  TGA  CCA  CCA  TGC  TCA  GCC  TCT  GTG  GAG  GTG  AAG  ACG  ACA  TTG  AGG
     ACT  GGT  GGT  ACG  AGT  CGG  AGA  CAC  CTC  CAC  TTC  TGC  TGT  AAC  TCC


 91  CCG  ACC  ACG  TAG  GCA  CCT  ATG  GTA  TAA  GTG  TAT  ATC  AGT  CTC  CTG
     GGC  TGG  TGC  ATC  CGT  GGA  TAC  CAT  ATT  CAC  ATA  TAG  TCA  GAG  GAC


136  GAG  ACA  TTG  GCC  AGT  ACA  CAT  TTG  AAT  TTG  ATG  GTG  ATG  AGT  TGT
     CTC  TGT  AAC  CGG  TCA  TGT  GTA  AAC  TTA  AAC  TAC  CAC  TAC  TCA  ACA


181  TCT  ATG  TGG  ACT  TGG  ATA  AGA  AGG  AGA  CTG  TCT  GGA  TGC  TTC  CTG
     AGA  TAC  ACC  TGA  ACC  TAT  TCT  TCC  TCT  GAC  AGA  CCT  ACG  AAG  GAC


226  AGT  TTG  GCC  AAT  TGG  CAA  GCT  TTG  ACC  CCC  AAG  GTG  GAC  TGC  AAA
     TCA  AAC  CGG  TTA  ACC  GTT  CGA  AAC  TGG  GGG  TTC  CAC  CTG  ACG  TTT


271  ACA  TAG  CTG  TAG  TAA  AAC  ACA  ACT  TGG  GAG  TCT  TGA  CTA  AGA  GGT
     TGT  ATC  GAC  ATC  ATT  TTG  TGT  TGA  ACC  CTC  AGA  ACT  GAT  TCT  CCA


316  CAA  ATT  CCA  CCC  CAG  CTA  CCA  ATG  AGG  CTC  CTC  AAG  CGA  CTG  TGT
     GTT  TAA  GGT  GGG  GTC  GAT  GGT  TAC  TCC  GAG  GAG  TTC  GCT  GAC  ACA


361  TCC  CCA  AGT  CCC  CTG  TGC  TGC  TGG  GTC  AGC  CCA  ACA  CCC  TCA  TCT
     AGG  GGT  TCA  GGG  GAC  ACG  ACG  ACC  CAG  TCG  GGT  TGT  GGG  AGT  AGA
```

EP 0 630 255 B1

FIG. 8-1

406 GCT TTG TGG ACA ACA TCT TCC CTC CTG TGA TCA ACA TCA CAT GGC
    CGA AAC ACC TGT TGT AGA AGG GAG GAC ACT AGT TGT AGT GTA CCG

451 TCA GAA ARA GCA AGT CAG TCG CAG ACG GTG TTT ATG AGA CCA GCT
    AGT CTT TYT CGT TCA GTC AGC GTC TGC CAC AAA TAC TCT GGT CGA

496 TCT TCG TCA ACC GTG ACT ATT CCT TCC ACA AGC TGT CTT ATC TCA
    AGA AGC AGT TGG CAC TGA TAA GGA AGG TGT TCG ACA GAA TAG AGT

541 CCT TCA TCC CTT CTG ACG ATG ACA TTT ATG ACT GCA AGG TGG AAC
    GGA AGT AGG GAA GAC TGC TAC TGT AAA TAC TGA CGT TCC ACC TTG

586 ACT GGG GCC TGG AGG AGC CGG TTC TGA AAC ACT GGG AAC CTG AGA
    TGA CCC CGG ACC TCC TCG GCC AAG ACT TTG TGA CCC TTG GAC TCT

631 TTC CAG CCC CCA TGT CAG AGC TGA CAG AGA CTG TGG TGT GTG CCC
    AAG GTC GGG GGT ACA GTC TCG ACT GTC TCT GAC ACC ACA CAC GGG

676 TGG GGT TGT CTG TGG GCC TTG TGG GCA TCG TGG TGG GCA CCA TCT
    ACC CCA ACA GAC ACC CGG AAC ACC CGT AGC ACC ACC CGT GGT AGA

721 TCA TCA TTC AAG GCC TGC GAT CAG GTG GCA CCT CCA GAC ACC CAG
    AGT AGT AAG TTC CGG ACG CTA GTC CAC CGT GGA GGT CTG TGG GTC

766 GGC CTT TAT GA
    CCG GAA ATA CT

**FIG. 8-2**

EP 0 630 255 B1

IAB BETA CHAIN

CAT TTC GTG TAC CAG TTC ATG GGC GAG TGC TAC TTC ACC AAC GGG
GTA AAG CAC ATG GTC AAG TAC CCG CTC ACG ATG AAG TGG TTG CCC

ACG CAG CGC ATA CGA TAT GTG ACC AGA TAC ATC TAC AAC CGG GAG
TGC GTC GCG TAT GCT ATA CAC TGG TCT ATG TAG ATG TTG GCC CTC

GAG TAC GTG CGC TAC GAC AGC GAC GTG GGC GAG CAC CGC GCG GTG
CTC ATG CAC GCG ATG CTG TCG CTG CAC CCG CTC GTG GCG CGC CAC

ACC GAG CTG GGG CGG CCA GAC GCC GAG TAC TGG AAC AGC CAG CCG
TGG CTC GAC CCC GCC GGT CTG CGG CTC ATG ACC TTG TCG GTC GGC

GAG ATC CTG GAG CGA ACG CGG GCC GAG CTG GAC ACG GTG TGC AGA
CTC TAG GAC CTC GCT TGC GCC CGG CTC GAC CTG TGC CAC ACG TCT

CAC AAC TAC GAG GGG CCG GAG ACC CAC ACC TCC CTG CGG CGG CTT
GTG TTG ATG CTC CCC GGC CTC TGG GTG TGG AGG GAC GCC GCC GAA

GAA CAG CCC AAT GTC GTC ATC TCC CTG TCC AGG ACA GAG GCC CTC
CTT GTC GGG TTA CAG CAG TAG AGG GAC AGG TCC TGT CTC CGG GAG

AAC CAC CAC AAC ACT CTG GTC TGC TCA GTG ACA GAT TTC TAC CCA
TTG GTG GTG TTG TGA GAC CAG ACG AGT CAC TGT CTA AAG ATG GGT

EP 0 630 255 B1

*FIG. 9-1*

EP 0 630 255 B1

```
361 GCC AAG ATC AAA GTG CGC TGG TTC CGG AAT GGC CAG GAG GAG ACG
    CGG TTC TAG TTT CAC GCG ACC AAG GCC TTA CCG GTC CTC CTC TGC

406 GTG GGG GTC TCA TCC ACA CAG CTT ATT AGG AAT GGG GAC TGG ACC
    CAC CCC CAG AGT AGG TGT GTC GAA TAA TCC TTA CCC CTG ACC TGG

451 TTC CAG GTC CTG GTC ATG CTG GAG ATG ACC CCT CGG CGG GGA GAG
    AAG GTC CAG GAC CAG TAC GAC CTC TAC TGG GGA GCC GCC CCT CTC

496 GTC TAC ACC TGT CAC GTG GAG CAT CCC AGC CTG AAG AGC CCC ATC
    CAG ATG TGG ACA GTG CAC CTC GTA GGG TCG ACT TTC TCG GGG TAG

541 ACT GTG GAG TGG AGG GCA CAG TCT GAG TCT GCC TGG AGC AAG ATG
    TGA CAC CTC ACC TCC CGT GTC AGA CTC AGA CGG ACC TCG TTC TAC

586 TTG AGC GGC ATC GGG GGC TGC GTG CTT GGG GTG ATC TTC CTC GGG
    AAC TCG CCG TAG CCC CCG ACG CAC GAA CCC CAC TAG AAG GAG CCC

631 CTT GGC CTT TTC ATC CGT CAC AGG AGT CAG AAA GGA CCT CGA GGC
    GAA CCG GAA AAG TAG GCA GTG TCC TCA GTC TTT CCT GGA GCT CCG

676 CCT CCT CCA GCA GGG CTC CTG CAG TGA
    GGA GGA GGT CGT CCC GAG GAC GTC ACT
```

FIG. 9-2

| # | HPTYP FREQ%[2] | DQ | DQBI | DQAI[3] | DRBI | DRB3 | DRB4 | D | DISEASE ASSOCIATION[4] |
|---|---|---|---|---|---|---|---|---|---|
| 1. | 20 | w5(wl) | 1.1 | la | 1 | ne | ne | wl | IDDM*, RA† |
| 2. | | w5(wl) | 1.1 | la | 1 | ne | ne | w20 | IDDM |
| 3. | 26 | w6(wl) | 1.2 | lb | wl5(2) | ne | ne | w2 | CPMS, MG(T+) |
| 4. | 1.5 | w6(wl) | 1.12 | lc | wl5(2) | ne | ne | wl2 | |
| 5. | 1.5 | w5(wl) | 1.1 | ? | wl6(2) | ne | ne | w21(AZH) | IDDM, MG(T-) |
| 6. | ? | w7(w3) | 3.1 | ? | wl6(2) | ne | ne | w22 | |
| 7. | 22 | w2 | ? | ? | wl7(3) | 24(52) | ne | w3 | |
| 8. | | w2 | ? | ? | wl7(3) | 25(52) | ne | w3 | IDDM, MG(T-) |
| 9. | ? | w4(Wa) | Wa | ? | wl8(3) | ?(52) | ne | ? | |
| 10. | 9 | w8(w3) | 3.2 | 3 | 4 | ne | 53 | w4(4.2) | IDDM*, RA†, CPMS |
| 11. | 5 | w7(w3) | 3.1 | 3 | 4 | ne | 53 | w4(4.1) | |
| 12. | 3 | w8(w3) | 3.2 | 3 | 4 | ne | 53 | wl0 | IDDM*, CPMS |
| 13. | ? | w7(w3) | 3.1 | 3 | 4 | ne | 53 | wl3 | |
| 14. | 14 | w8(w3) | 3.2 | 3 | 4 | ne | 53 | wl4 | IDDM*, RA†, CPMS |
| 15. | 0.5 | w4(Wa) | Wa | ? | 4 | ne | 53 | wl5 | RA |

FIG. 10-1

EP 0 630 255 B1

| | | | | | | | | | |
|---|---|---|---|---|---|---|---|---|---|
| 16. | 15 | ⎡w7(w3) | 3.1 | 2 | wII(5) | 25(52) | ne | w5⎤ | MG |
| 17. | | ⎣w7(w3) | 3.1 | 2 | wI2(5) | 25(52) | ne | B6⎦ | |
| 18. | 10 | ⎡w5(wI) | 1.18 | Ic | w(13)(w6) | 24(52) | ne | wI8 | |
| 19. | | ⎣w5(wI) | 1.18 | Ic | w(13)(w6) | 25(52) | ne | wI8 | |
| 20. | 3 | w5(wI) | 1.19 | Ib | w(13)(w6) | 26(52) | ne | wI9 | IDDM |
| 21. | 3 | w6(wI) | 1.9 | Ia | w(14)(w6) | 25(52) | ne | w9 | |
| 22. | ? | w6(wI) | 1.16 | 2 | w(14)(w6) | 24(52) | ne | wI6 | RA |
| 23. | 1 | w9(w3) | 3.3 | 3 | 7 | ne | 53 | wII | |
| 24. | 27 | w2 | 2 | 3 | 7 | ne | 53 | wI7 | |
| 25. | 6 | w4(Wa) | Wa | Ib | ne | w8/52 | ne | w8 | |
| 26. | 2 | ?(w3) | ? | Ib | ne | w8/52 | ne | w8 | |
| 27. | 1 | w9(w3) | 3.3 | 3 | 9 | ne | 53 | w23 | |
| 28. | ? | w5(wI) | 1.1 | Ia | wI0 | ? | ? | ? | |

FIG. 10-2

EP 0 630 255 B1

PATIENT PRESENTS

AUTOIMMUNE DISEASE IDENTIFIED

MHC II TYPING UNDERTAKEN

CELL TYPING
BY MLR(HTC)

SEROLOGIC

DNA (PCR WITH
PBL DNA)

PATIENT MHC II HAPLOTYPES DEFINED

IN VITRO DETERMINATION OF AUTOPEPTIDE(S)
RECOGNIZED BY PATIENT AUTOREACTIVE T CELLS IN
ASSOCIATION WITH THE APPROPRIATE
MHC II HAPLOTYPE REAGENT

INITIATE THERAPY WITH TOXIN CONJUGATE OF
APPROPRIATE PEPTIDE·MHC II HAPLOTYPE

MONITOR THERAPY BY PATIENT T CELL BINDING STUDIES
USING THE SAME PEPTIDE-MHC II HAPLOTYPE
WITH ATTACHED REPORTER GROUP

*FIG. II.*

100 C3H/HeN MICE, 8-9 WEEKS OLD     CH$_{27}$ CELLS = 1 X 10$^{10}$
TOTAL SPLEEN CELLS = 4.5-6.0X10$^9$

CULTURED FOR 36 HOURS AT 3X10$^6$ CELLS/ml
WITH 100 μg/ml LPS AND 5% FCS

LYSED CELLS AND HOMOGENIZED IN
10mM TRIS HCl pH 8.3, ImM PMSF .02% NaN$_3$
(FOR 5X10$^9$ CELLS, 50ml BUFFER WAS USED)

CENTRIFUGED FOR 15 MIN. AT
2,000 Xg AT 4°C
PELLET   TO REMOVE CELL DEBRIES
SUPERNATANT

CENTRIFUGED FOR 60 MIN. AT
SUPERNATANT   100,000Xg AT 4°C
PELLET

SUSPENDED IN DNase 1 BUFFER
(10mM TRIS HCl pH 8.3, ImM EDTA, 0.5mg/ml DNase 1)
FOR 5X10$^9$ CELLS, 10ml BUFFER WAS USED

25°C FOR 1 HOUR WITH GENTLE
CONSTANT SHAKING

ADDED HIGH SALT/EDTA BUFFER
(10mM TRIS pH 8.3, 0.5 M NaCl, 5mM EDTA,
ImM PMSF AND .02% NaN$_3$)
FOR 5X10$^9$ CELLS, 50 ml BUFFER WAS USED

4°C FOR 30 MIN. WITH GENTLE
SHAKING CENTRIFUGED FOR 60
MIN. AT 100,000Xg AT 4°C
SUPERNATANT
PELLET

SUSPENDED IN NP-40 BUFFER
(10mM TRIS HCl pH8.3, 0.5% NP40,
0.1 M NaCl, 5mM EDTA, 0.02% NaN$_3$ AND ImM PMSF)
FOR 5X10$^9$ CELLS, 50 ml BUFFER WAS USED

CENTRIFUGE FOR 60 MIN. AT
PELLET   100,000 X g AT 4°C

SUPERNATANT

(TOTAL PROTEIN = 18-23 mg)

*FIG. 12.*

10-2.16  Mob  WERE  PURIFIED  ON  PROTEIN-A  COLUMN
FROM  5  ml  ASCITES, 30mg  PURE  Ab  WERE  RECOVERED.
A  STANDARD  PROCEDURE  (MAPS II)  FROM  Bio-Rad  LABORATORIES  WAS  FOLLOWED

$\downarrow$

COUPLED  TO  CNBr  ACTIVATED  SEPHAROSE  4B

(3-4 mg Ab/ml  OF  WET  GEL)

STANDARD  PROCEDURE  FROM  PHARMACIA  WAS  FOLLOWED

EXTENT  OF  COUPLING $\Rightarrow$ 98%

$\downarrow$

PURIFIED  IgG  WAS  ANALYZED  ON  10%  ID  POLYACRYLAMIDE  GEL
FOLLOWED  BY  COOMASSIE  BRILLIANT  BLUE  R-250

*FIG. 13a.*

EP 0 630 255 B1

EP 0 630 255 B1

ONE DIMENSIONAL POLYACRYLAMIDE
GEL ANALYSIS OF PURIFIED 10 - 2.16
MONOCLONAL ANTIBODY

FIG. 13b.

ONE DIMENSIONAL POLYACRLAMIDE
GEL ANALYSIS OF IAk

FIG. 15.

41

WASHED 10-2.16 ANTIBODY COLUMN WITH LOW AND HIGH pH
BUFFERS AND EQUILIBRATED WITH 0.5% NP-40 BUFFER, pH
8.3 (10mM TRIS HCl pH 8.3, 0.5% NP40, 0.1 M NaCl,
5 mM EDTA, 0.02% NaN$_3$ AND 1mM PMSF)

$\downarrow$

APPLIED 50ml MEMBRANE FRACTION (20-25mg TOTAL PROTEIN)
TO A 4ml BED VOLUME COLUMN CONTAINING 15-20mg
COUPLED ANTIBODIES (CIRCULATED OVERNIGHT AT 4°C)

$\downarrow$

WASHED COLUMN WITH 10 BED VOLUMES OF
0.5% DOC BUFFER, pH 8.3
(1mM TRIS HCl, pH 8.3, 0.5% DEOXYCHOLATE, 0.1 M NaCl,
5mM EDTA. 0.02% NanN$_3$ AND 5mM PMSF)

$\downarrow$

WASHED WITH 5 BED VOLUMES OF
1% OG BUFFER IN PHOSPHATE BUFFER, pH 8.3
(20mM PHOSPHATE pH 8.3, 0.1 M NaCl, 1% OCTYLGLUCOSIDE
.02% NaN$_3$ AND 5mM PMSF)

$\downarrow$

ELUTED WITH 1% OG IN PHOSPHATE BUFFER, pH 11.0
(20mM PHOSPHATE pH11, 0.1 M NaCl, 1% OCTYLGLUCOSIDE
.02% NaN$_3$ AND 5 mM PMSF)
NEUTRALIZED EACH 1ml FRACTION WITH 12 μl OF 1 M ACETIC ACID

$\downarrow$

POOLED PEAK WAS CONCENTRATED (5-10 FOLD)
BY VACUUM DIALYSIS

$\downarrow$

TOTAL 1-A$^k$ =200-300 μg (FROM 5X10$^9$ SPLEEN CELLS)
ANALYZED ON 10% PAGE FOLLOWED BY SILVERSTAIN

## FIG. 14.

FIG. 16.

FIG. 17.

FIG. 18.

FIG. 19A.

FIG. 19B.

FIG. 19C.

9r139

SEPHADEX G-200 CHROMATOGRAPHY
OF DETERGENT FREE PURIFIED IAk

Fig. 20

46